# EUROPEAN PATENT APPLICATION

(11) **EP 3 932 426 A2**
(43) Date of publication of application: **05.01.2022**
(21) Application number: 20763678.8
(22) Date of filing: 25.02.2020
(51) Int. Cl.: A61K 39/395, C07K 16/46, A61P 35/00

(54) **PREPARATIONS CONTAINING ANTI-CD47 ANTIBODY, AND PREPARATION METHOD AND USE THEREFOR**

(30) Priority: 26.02.2019 CN 201910141894
(71) Applicant: Innovent Biologics (Suzhou) Co., Ltd., Suzhou, Jiangsu 215123 (CN)
(72) Inventor: XIE, Ruixia, Suzhou, Jiangsu 215123 (CN); MA, Liqiang, Suzhou, Jiangsu 215123 (CN); WANG, Yinjue, Suzhou, Jiangsu 215123 (CN); ZHOU, Kaisong, Suzhou, Jiangsu 215123 (CN)
(74) Representative: Ruscoe, Peter James
(86) International application number: PCT/CN2020/076611
(87) International publication number: WO 2020/173431

(57) **Abstract**

The present invention relates to a formulation comprising an anti-CD47 antibody, and in particular to a pharmaceutical formulation comprising an antibody, a buffer, a stabilizer and a surfactant. Furthermore, the present invention also relates to therapeutic or prophylactic use of those formulations.

## Description

### TECHNICAL FIELD

The present invention relates to the field of antibody formulations. Specifically, the present invention relates to a pharmaceutical formulation comprising a recombinant fully human anti-cluster of differentiation 47 (CD47) monoclonal antibody, and in particular to a stable high-concentration antibody liquid formulation, a method for preparing the pharmaceutical formulation, and therapeutic and/or prophylactic use of the pharmaceutical formulation.

### BACKGROUND

Cluster of differentiation 47 (CD47), also known as an integrin-associated protein (IAP), is a member of the immunoglobulin superfamily. Different studies have shown that almost all tumor cells and tissues can highly express CD47. Multiple anti-CD47 antibodies have been reported to be developed for the treatment of various tumors and/or cancers.

A series of recombinant fully human anti-cluster of differentiation 47 (CD47) monoclonal antibodies are disclosed in Chinese Application No. CN201710759828.9 (filed on Aug. 29, 2017). Those antibodies can improve the phagocytosis of macrophages and have remarkable anti-tumor activity, which can significantly inhibit the tumor growth and even completely eliminate the tumors. In addition, those antibodies also exhibit significantly reduced hemagglutination, resulting in significantly reduced side effects in clinical treatment.

Monoclonal antibodies are highly specific to the target and are generally more effective than small molecule drugs. However, the development of monoclonal antibody-based drugs has its own challenges. Monoclonal antibodies are more complex than traditional organic and inorganic drugs and often have a similar degradation pattern to other protein biologics. Broadly, the degradation of antibody proteins can be classified into two main types: physical instability (involving changes in the higher order structure of the protein) and chemical instability (involving various chemical modifications of the protein). Chemical instability can be caused by deamidation, racemization, hydrolysis, oxidation, β-elimination, disulfide bond exchange or the like, the most common of which are fragmentation, deamidation and oxidation. Physical instability can result from denaturation, aggregation, precipitation, adsorption or the like.

The degradation of monoclonal antibodies can occur at various stages, including the preparation, storage, and delivery of antibody formulations. The biological activity of monoclonal antibody-based drugs is closely related to their structure, conformation and chemical stability. Accordingly, the development of antibody formulations is an important aspect for the development of antibody products and is often a key step in successful clinical production. The stability of antibody drugs is an important index to ensure the efficacy and safety of drugs. It is a key condition for a drug to maintain its efficacy and safety over the shelf life to obtain a formulation prescription that confers good stability to an antibody drug. Accordingly, the antibody formulation must be formulated in a manner that not only makes the antibody suitable for administration to a subject, but also maintains its stability during storage and subsequent use. If an antibody is not properly formulated in a liquid, the antibody in the liquid solution is prone to decomposition, aggregation, undesirable chemical modification or the like.

Monoclonal antibodies are currently being developed for the treatment of various indications at increased doses. For example, cetuximab is administered at a dose of 250-400 mg/m²; efalizumab is administered at a dose of about 1 mg/kg. In addition, there is an increasing demand for subcutaneous injection of antibody formulations taking the convenience of administration and patient compliance into account. Accordingly, it is one direction in this field to develop stable high-concentration monoclonal antibody liquid formulations with the protein concentration needed to be 100 mg/mL or more.

In addition to meeting quality requirements such as stability and purity, flexibility of antibody formulations is also a factor considered in the biomedical field. Advantageously, the dosage form allows flexibility of administration over a wide range of dosage levels (typically, 0.1-20 mg/kg for monoclonal antibodies, depending on the indication of interest) as well as by a variety of routes of administration (typically, subcutaneous and intravenous administrations).

Accordingly, there is a need in the art for sufficiently stable new pharmaceutical formulations containing anti-CD47 antibodies, in particular for stable liquid formulations suitable for the antibodies at high concentrations (e.g., about 100 mg/mL).

### BRIEF SUMMARY

In order to develop a formulation prescription for long-term stable storage of a recombinant fully human anti-CD47 monoclonal antibody, and to ensure that the quality of the product is controllable over its period of validity (e.g., at least 24 months), the present inventors design tests of two stages of pre-prescription and prescription screening to investigate the effects of different pH, surfactants, stabilizers, and antioxidants on the stability of anti-CD47 antibody formulations. Through this intensive study, the present inventors provide a pharmaceutical formulation comprising a recombinant fully human anti-cluster of differentiation 47 (CD47) monoclonal antibody, in particular a stable high-concentration antibody liquid formulation.
In one aspect, the present invention provides a liquid antibody formulation comprising (i) a recombinant fully human anti-CD47 monoclonal antibody (hereinafter also referred to as "anti-CD47 antibody"), (ii) a buffer, (iii) a stabilizer, and (iv) a surfactant.
In one embodiment, the anti-CD47 antibody is a recombinant fully human anti-cluster of differentiation 47 (CD47) monoclonal antibody disclosed in Chinese Application No. CN201710759828.9 (filed on Aug. 29, 2017), the content of which is incorporated herein by reference in its entirety for the purpose of the present application. In one embodiment, the anti-CD47 antibody comprises a heavy chain variable region and a light chain variable region, wherein the heavy chain variable region comprises a sequence of SEQ ID NO: 1 or a sequence having at least 90% identity thereto, and the light chain variable region comprises a sequence of SEQ ID NO: 2 or a sequence having at least 90% identity thereto: 
QVQLQESGPGLVKPSETLSLTCTVSGGSISSYYWSWIRQPPGKGLEWIGYIYYSGSTNYNPSLKSRVT
ISVDTSKNQFSLKLSSVTAADTAVYYCARGKTGSAAWGQGTLVTVSS (SEQ ID NO: 1);
 
DIQMTQSPSSVSASVGDRVTITCRASQGISRWLAWYQQKPGKAPKLLIYAASSLQSGVPSRFSGSGS
GTDFTLTISSLQPEDFATYYCQQTVSFPITFGGGTKVEIK (SEQ ID NO: 2).
 In one embodiment, the anti-CD47 antibody comprises:
- heavy chain VH CDR1 of GSISSYYWS (SEQ ID NO: 3) or SYYWS (SEQ ID NO: 11);
- heavy chain VH CDR2 of YIYYSGSTNYNPSLKS (SEQ ID NO: 4);
- heavy chain VH CDR3 of ARGKTGSAA (SEQ ID NO: 5) or GKTGSAA (SEQ ID NO: 12);
- light chain VL CDR1 of RASQGISRWLA (SEQ ID NO: 6);
- light chain VL CDR2 of AASSLQS (SEQ ID NO: 7); and
- light chain VL CDR3 of QQTVSFPIT (SEQ ID NO: 8).
In one embodiment, the anti-CD47 antibody is an IgG4 antibody comprising a heavy chain and a light chain, wherein the heavy chain comprises a sequence of SEQ ID NO: 9 or a sequence having at least 90% identity thereto, and the light chain comprises a sequence of SEQ ID NO: 10 or a sequence having at least 90% identity thereto: 
QVQLQESGPGLVKPSETLSLTCTVSGGSISSYYWSWIRQPPGKGLEWIGYIYYSGSTNYNPSLKSRVT
ISVDTSKNQFSLKLSSVTAADTAVYYCARGKTGSAAWGQGTLVTVSSASTKGPSVFPLAPCSRST
SESTAALGCLVKDYFPEPVTVSWNSGALTSGVHTFPAVLQSSGLYSLSSVVTVPSSSLGTKT
YTCNVDHKPSNTKVDKRVESKYGPPCPPCPAPEAAGGPSVFLFPPKPKDTLMISRTPEVTCV
VVDVSQEDPEVQFNWYVDGVEVHNAKTKPREEQFNSTYRVVSVLTVLHQDWLNGKEYK
CKVSNKGLPSSIEKTISKAKGQPREPQVYTLPPSQEEMTKNQVSLTCLVKGFYPSDIAVEWE
 
SNGQPENNYKTTPPVLDSDGSFFLYSRLTVDKSRWQEGNVFSCSVMHEALHNHYTQKSLS
LSLG (SEQ ID NO: 9)
 
DIQMTQSPSSVSASVGDRVTITCRASQGISRWLAWYQQKPGKAPKLLIYAASSLQSGVPSRFSGSGS
GTDFTLTISSLQPEDFATYYCQQTVSFPITFGGGTKVEIKRTVAAPSVFIFPPSDEQLKSGTASVVC
LLNNFYPREAKVQWKVDNALQSGNSQESVTEQDSKDSTYSLSSTLTLSKADYEKHKVYAC
EVTHQGLSSPVTKSFNRGEC (SEQ ID NO: 10).
 Preferably, the anti-CD47 antibody is the anti-CD47 monoclonal antibody ADI-26630 disclosed in Chinese Application No. CN201710759828.9 (filed on Aug. 29, 2017), which consists of a heavy chain sequence of SEQ ID NO: 9 and a light chain sequence of SEQ ID NO: 10. In a mouse tumor model in which human burkitt's lymphoma cells are transplanted, the antibody exhibits a significant anti-tumor activity, for example, intraperitoneal injection of 5 mg/kg antibody once every two days for two consecutive weeks can result in a tumor growth inhibition of about 100% or more; and the tumor disappearance rate can reach 60% or more.

In one embodiment, the concentration of the anti-CD47 antibody in the liquid antibody formulation is about 1-150 mg/mL, e.g., 20 mg/mL or more, particularly 50 mg/mL or more, preferably 100 mg/mL or 120 mg/mL. For example, the concentration of the anti-CD47 antibody in the liquid antibody formulation may be about 15 mg/mL, 20 mg/mL, 25 mg/mL, 30 mg/mL, 35 mg/mL, 40 mg/mL, 45 mg/mL, 50 mg/mL, 55 mg/mL, 60 mg/mL, 70 mg/mL, 75 mg/mL, 80 mg/mL, 85 mg/mL, 90 mg/mL or more, e.g., 90 mg/mL, 95 mg/mL, 100 mg/mL, 105 mg/mL, 110 mg/mL or 120 mg/mL.
In one embodiment, the liquid antibody formulation disclosed herein is at pH 5.0-6.0, e.g., at pH 5.2 ± 0.2, pH 5.5 ± 0.2 or pH 5.7 ± 0.2, preferably at pH 5.5.
In one embodiment, the liquid antibody formulation disclosed herein comprises about 5-100 mM buffer. In one embodiment, the concentration of the buffer in the liquid antibody formulation disclosed herein is about 10 mM, 20 mM, 30 mM, 40 mM or 50 mM. In one embodiment, the buffer is a histidine buffer. In a preferred embodiment, the liquid antibody formulation disclosed herein comprises about 10-20 mM histidine buffer, particularly 10 mM histidine buffer. In another embodiment, the histidine buffer consists of a histidine-histidine hydrochloride buffer system. In another preferred embodiment, the liquid antibody formulation disclosed herein comprises 0.4 mg/mL histidine and 1.5 mg/mL histidine hydrochloride.
In one embodiment, the liquid antibody formulation disclosed herein comprises a stabilizer, preferably, the stabilizer is selected from sorbitol, sucrose, trehalose, arginine and a combination thereof, more preferably sorbitol or a combination of sorbitol and arginine. In a preferred embodiment, the liquid formulation disclosed herein comprises sorbitol as a stabilizer, preferably sorbitol at a concentration of about 1-10% w/v, e.g., 1.5% w/v, 2% w/v, 2.5% w/v, 3% w/v, 3.5% w/v, 4% w/v, 4.5% w/v, 5% w/v, 5.5% w/v or 6% w/v, or in an amount of about 10-60 mg/mL, e.g., about 15 mg/mL, 20 mg/mL, 25 mg/mL, 30 mg/mL, 35 mg/mL, 40 mg/mL, 45 mg/mL, 50 mg/mL or 55 mg/mL, preferably about 40 mg/mL. In another preferred embodiment, the liquid antibody formulation disclosed herein comprises a stabilizer combination of sorbitol and arginine. Preferably, in the combination, the amount of sorbitol is about 10-30 mg/mL, particularly 15 mg/mL, and the amount of arginine is 80-110 mM, particularly about 100 mM. In another preferred embodiment, the liquid antibody formulation disclosed herein comprises a stabilizer combination of about 15 mg/mL sorbitol and about 21.1 mg/mL arginine hydrochloride.
In one embodiment, the liquid antibody formulation disclosed herein comprises a surfactant, preferably, the surfactant is a non-ionic surfactant. In one embodiment, the surfactant is selected from polysorbate surfactants, preferably polysorbate-20. In one embodiment, the liquid antibody formulation disclosed herein comprises about 0.1-1 mg/mL, e.g., 0.1-0.5 mg/mL polysorbate-20. In a preferred embodiment, the liquid antibody formulation disclosed herein comprises about 0.1-0.4 mg/mL polysorbate-20. In another preferred embodiment, the amount of polysorbate-20 in the liquid antibody formulation disclosed herein is about 0.3 mg/mL.

In one embodiment, the liquid formulation disclosed herein also comprises an antioxidant. In another embodiment, the liquid formulation disclosed herein does not comprise an antioxidant. In one embodiment, the antioxidant is edetic acid (EDTA) or a salt thereof, e.g., EDTA-2Na salt.

In one embodiment, the liquid formulation is a stable liquid pharmaceutical formulation, preferably an injection. In one embodiment, the pharmaceutical formulation disclosed herein is for use in subcutaneous, intradermal, intramuscular, or intravenous injection.

In one preferred embodiment, the liquid antibody formulation disclosed herein comprises:
(i) about 10-150 mg/mL anti-CD47 antibody;
(ii) about 5-50 mM histidine buffer;
(iii) about 1-10% w/v sorbitol, or a stabilizer combination of about 1-5% w/v sorbitol and about 80-110 mM arginine; and
(iv) about 0.01-0.1% w/v polysorbate-20;
wherein the liquid formulation is at about pH 5.0-6.0, preferably at pH 5.5 ± 0.2.
In one preferred embodiment, the liquid antibody formulation disclosed herein comprises:
(i) 90-150 mg/mL, particularly 90-120 mg/mL, anti-CD47 antibody;
(ii) about 10-20 mM histidine buffer;
(iii) about 20-60 mg/mL (e.g., 40-50 mg/mL) sorbitol, or a combination of about 5-40 mg/mL (e.g., 10-20 mg/mL) sorbitol and about 80-110 mM (e.g., about 100 mM) arginine; and
(iv) about 0.1-0.4 mg/mL polysorbate-20;
wherein the liquid formulation is at about pH 5.0-6.0, preferably at pH 5.5 ± 0.2.
In one preferred embodiment, the liquid antibody formulation disclosed herein comprises:
(i) about 100 mg/mL anti-CD47 antibody;
(ii) about 0.4 mg/mL histidine and about 1.5 mg/mL histidine hydrochloride;
(iii) about 15.0 mg/mL sorbitol and about 21.1 mg/mL arginine hydrochloride; and
(iv) about 0.3 mg/mL polysorbate-20;
wherein the liquid formulation is at about pH 5.5.

In another aspect, the present invention provides a solid antibody formulation obtained by curing the liquid antibody formulation disclosed herein. The curing treatment is implemented by, e.g., crystallization, spray drying, or freeze drying. In a preferred embodiment, the solid antibody formulation is, e.g., in the form of a lyophilized formulation, e.g., a lyophilized powder for injection. The solid antibody formulation can be reconstituted in a suitable vehicle prior to use to give the reconstituted formulation disclosed herein. The reconstituted formulation is also a liquid antibody formulation disclosed herein. In one embodiment, the suitable vehicle is selected from water for injection, organic solvents for injection (including but not limited to, oil for injection, ethanol, propylene glycol, and the like), and a combination thereof.

The liquid formulation disclosed herein can be stably stored for a long period of time, particularly at a high concentration (e.g., 50 mg/mL or more, preferably 90-150 mg/mL, e.g., 100 mg/mL or 120 mg/mL), for example, for at least 24 months or longer. In one embodiment, the liquid formulation disclosed herein can be stably stored at about -80 °C to about 45 °C, e.g., -80 °C, about -30 °C, about -20 °C, about 0 °C, about 5 °C, about 25 °C, about 35 °C, about 38 °C, about 40 °C, about 42 °C or about 45 °C for at least 10 days, at least 20 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, at least 36 months, or longer.
In one embodiment, the liquid formulation disclosed herein can be stably stored for at least 24 months. In another embodiment, the liquid formulation disclosed herein is stable at a temperature of at least 40 °C. In another embodiment, the liquid formulation disclosed herein remains stable at about 2-8 °C for at least 12 months, preferably at least 24 months. In one embodiment, the liquid formulation disclosed herein remains stable at room temperature or, e.g., about 25 °C for at least 3 months, preferably at least 6 months. In another embodiment, the liquid formulation disclosed herein remains stable at about 40 °C for at least 1 month. In another embodiment, the liquid formulation disclosed herein can remain stable with shaking at about 5-40 °C, e.g., 25 °C, for at least 1 day, e.g., 3 days or 5 days.
In one embodiment, the stability of the formulation can be indicated after storage by detecting changes in the appearance, visible particles, protein content, purity and/or charge variants of the formulation. In one embodiment, the stability of the liquid formulation disclosed herein can be tested in a high-temperature accelerated test, for example, after storage at 40 ± 2 °C for at least 1 week, 2 weeks or preferably 1 month, or after storage at 25 ± 2 °C for at least 1 month or 2 months. In one embodiment, the shaking stability of the liquid formulation disclosed herein is tested by a shaking test.
In one embodiment, the stability of the liquid formulation disclosed herein is visually inspected after storage, wherein the liquid formulation disclosed herein remains a clear, colorless or yellowish liquid in appearance, free of particles, floccules and precipitates. In one embodiment, no visible particles exist in the formulation upon visual inspection under natural light. In one embodiment, the stability of the liquid formulation disclosed herein is tested after storage by determining the change in protein content, wherein the change rate in protein content is no more than 20%, preferably no more than 10%, e.g., 7-8%, preferably no more than 5% relative to the initial value on day 0 of storage, as measured, for example, by the ultraviolet spectrophotometry (UV) method. In one embodiment, the stability of the liquid formulation disclosed herein is tested after storage by determining the change in turbidity of the liquid formulation disclosed herein, wherein the change is no more than 0.04, preferably no more than 0.03, more preferably no more than 0.02 relative to the initial value on day 0 of storage, as measured, for example, by the OD_{350 mm} method. In one embodiment, the stability of the liquid formulation disclosed herein is tested after storage by determining the change in purity of the liquid formulation disclosed herein, wherein the change in main peak purity is no more than 10%, e.g., no more than 5%, 4%, 3%, e.g., 1-2%, preferably no more than 1%, and preferably the increase in the aggregates is no more than 2%, preferably no more than 1%, 0.5% or 0.1% relative to the initial value on day 0 of storage, as measured by the size exclusion high performance liquid chromatography (SEC-HPLC) method. In one embodiment, the stability of the liquid formulation disclosed herein is tested after storage by determining the change in purity of the formulation disclosed herein, wherein the change in main peak purity is reduced by no more than 10%, e.g., no more than 5%, 4%, 3%, preferably no more than 2%, 1%, 0.5% or 0.1%, as measured by the non-reduced capillary electrophoresis-sodium dodecyl sulfate (CE-SDS) method. In one embodiment, the stability of the liquid formulation disclosed herein is tested after storage by imaging capillary isoelectric focusing (iCIEF), wherein the change in charge variants (principal component, acidic component or basic component) of the antibody is no more than 30%, preferably no more than 20% or 10%, e.g., no more than 5%, 4%, 3% or 2% relative to the initial value on day 0 of storage, for example, in one embodiment, the change in principal component or acidic component is no more than 20% and the change in basic component is no more than 3%.
In one embodiment, the formulation is stable after storage, e.g., at 2-8 °C for at least 24 months, at room temperature for at least 3 months, or at 40 ± 2 °C for 1 month, and preferably, has one or more of the following characteristics:
(i) the percentage of antibody monomers in the formulation is greater than 90%, preferably greater than 95%, and preferably, the increase in the aggregates is no more than 2%, as measured by SEC;
(ii) the formulation has a purity of greater than 90%, preferably greater than 95%, as measured by non-reduced CE-SDS; and
(iii) at least 50%, preferably at least 55% of the anti-CD47 antibody in the formulation is in non-basic and non-acidic forms relative to the initial value on day 0 of storage, and preferably, the increase in charge variant (acidic component) of the antibody is no more than 20%, as measured by iCIEF.
In another embodiment, the formulation is stable after storage, e.g., at 2-8 °C for at least 24 months, at room temperature for at least 3 months, or at 40 ± 2 °C for 1 month, and preferably, has one or more of the following characteristics:
(i) the appearance is clear, and no visible particles exist;
(ii) the change rate in protein content is no more than 10% relative to the initial value on day 0 of storage, as measured by the ultraviolet spectrophotometry (UV) method;
(iii) the change in turbidity is no more than 0.04, preferably no more than 0.02 relative to the initial value on day 0 of storage, as measured by the OD_{350 mm} method;
(iv) the change in main peak purity is no more than 5%, preferably no more than 1% relative to the initial value on day 0 of storage, as measured by SEC-HPLC;
(v) the change in main peak purity is reduced by no more than 5%, preferably no more than 3% relative to the initial value on day 0 of storage, as measured by non-reduced CE-SDS; and
(vi) at least 50%, preferably at least 55% of the anti-CD47 antibody in the formulation is in non-basic and non-acidic forms relative to the initial value on day 0 of storage, and preferably, the increase in charge variant (acidic component) of the antibody is no more than 20%, as measured by iCIEF.

In one aspect, the present invention provides a delivery device comprising the liquid antibody formulation or the solid antibody formulation disclosed herein. In one embodiment, the delivery device disclosed herein is provided in the form of a pre-filled syringe comprising the liquid antibody formulation or the solid antibody formulation disclosed herein, e.g., for use in intravenous, subcutaneous, intradermal or intramuscular injection.
In another aspect, the present invention provides a method for delivering the anti-CD47 antibody to a subject, e.g., a mammal, comprising administering the liquid antibody formulation or the solid antibody formulation disclosed herein to the subject, the delivery being implemented, e.g., using a delivery device of a pre-filled syringe.
In another aspect, the present invention provides use of the liquid antibody formulation or the solid antibody formulation disclosed herein in the preparation of a delivery device (e.g., a pre-filled syringe) or a medicament for treating CD47-related diseases in a subject, in particular for treating CD47-related cancers, e.g., various hematological tumors, such as lymphomas, e.g., burkitt's lymphoma.
Other embodiments of the present invention will become apparent by reference to the detailed description hereinafter.

### BRIEF DESCRIPTION OF THE DRAWINGS

The preferred embodiments of the present invention described in detail below will be better understood when read in conjunction with the following drawings. For the purpose of illustrating the present invention, currently preferred embodiments are shown in the drawings. However, it should be understood that the present invention is not limited to accurate arrangement and means of the embodiments shown in the drawings.

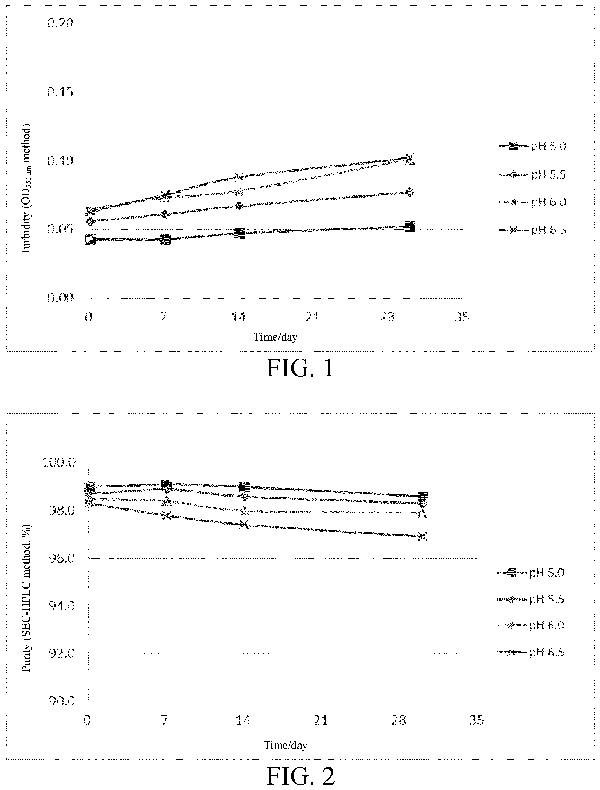

FIG. 1 is a graph showing the change in turbidity over time of the antibody formulations at various pH values (pH 5.0, 5.5, 6.0 and 6.5) after storage at 40 ± 2 °C for 0 days, 1 week, 2 weeks and 1 month as determined by the OD_{350 nm} method in the pH screening test described in the example.
FIG. 2 is a graph showing the change in main peak purity over time of the antibody formulations at various pH values (pH 5.0, 5.5, 6.0 and 6.5) after storage at 40 ± 2 °C for 0 days, 1 week, 2 weeks and 1 month as determined by the SEC-HPLC method in the pH screening test described in the example.

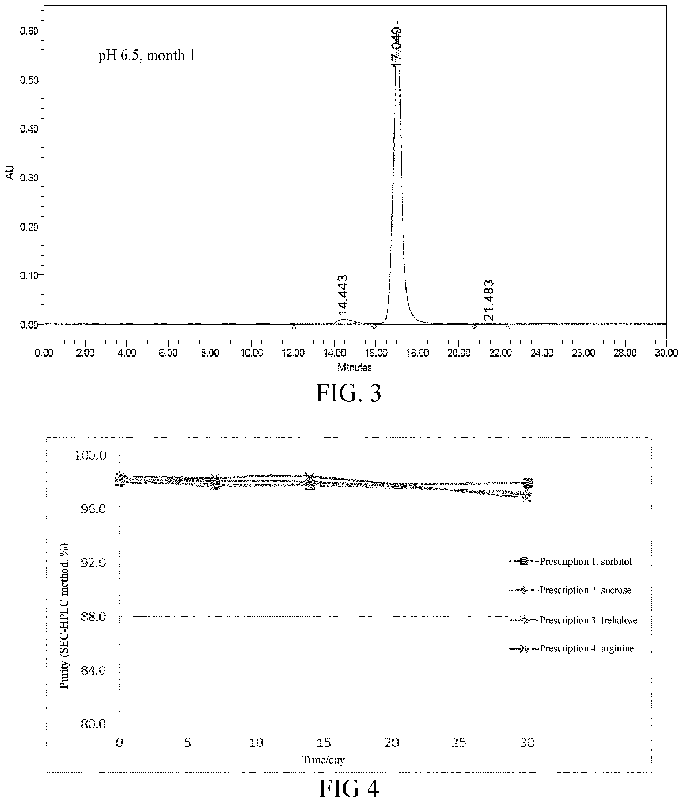

FIG. 3 shows the purity chromatogram of the antibody formulation at pH 6.5 after storage at 40 ± 2 °C for 1 month as determined by the SEC-HPLC method in the pH screening test described in the example.
FIG. 4 is a graph showing the change in main peak purity over time of the antibody formulations having different stabilizers (prescriptions 1 to 4) after storage at 40 ± 2 °C for 0 days, 1 week, 2 weeks and 1 month as determined by the SEC-HPLC method in the stabilizer screening test described in the example.

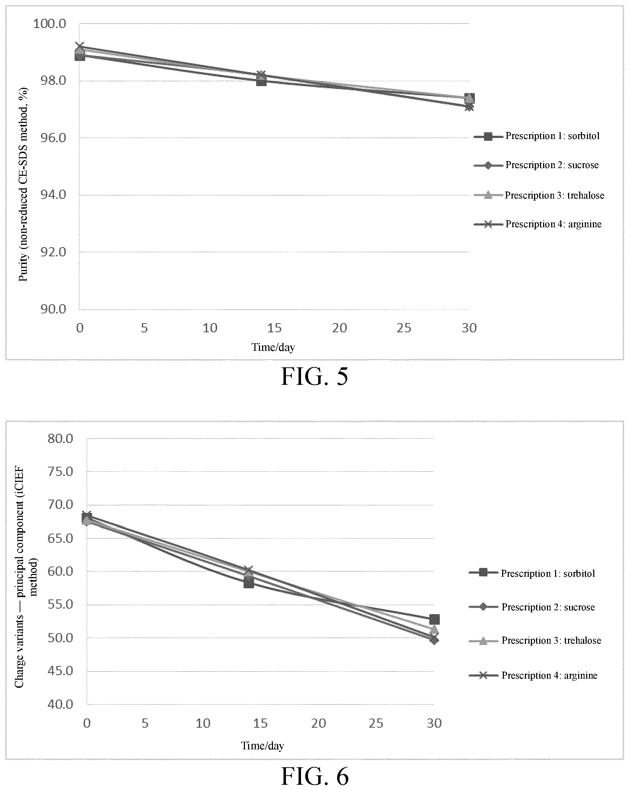

FIG. 5 is a graph showing the change in main peak purity over time of the antibody formulations having different stabilizers (prescriptions 1 to 4) after storage at 40 ± 2 °C for 0 days, 1 week, 2 weeks and 1 month as determined by the non-reduced CE-SDS method in the stabilizer screening test described in the example.
FIG. 6 is a graph showing the change in charge variant (principal component) over time of the antibody formulations having different stabilizers (prescriptions 1 to 4) after storage at 40 ± 2 °C for 0 days, 1 week, 2 weeks and 1 month as determined by the iCIEF method in the stabilizer screening test described in the example.

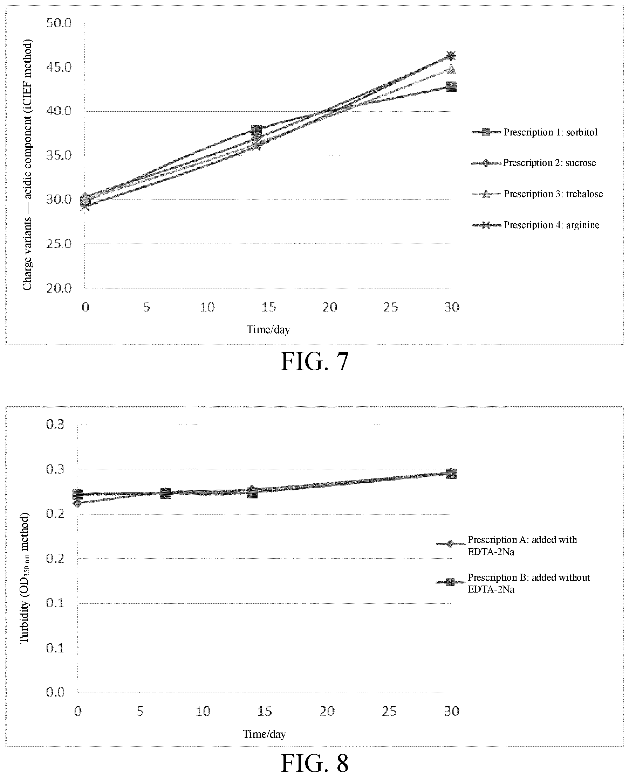

FIG. 7 is a graph showing the change in charge variant (acidic component) over time of the antibody formulations having different stabilizers (prescriptions 1 to 4) after storage at 40 ± 2 °C for 0 days, 1 week, 2 weeks, and 1 month as determined by the iCIEF method in the stabilizer screening test described in the example.
FIG. 8 is a graph showing the change in turbidity over time of the antibody formulations added with or without EDTA (prescriptions A and B) after storage at 40 ± 2 °C for 0 days, 1 week, 2 weeks and 1 month as determined by the OD_{350 nm} method in the antioxidant screening test described in the example.

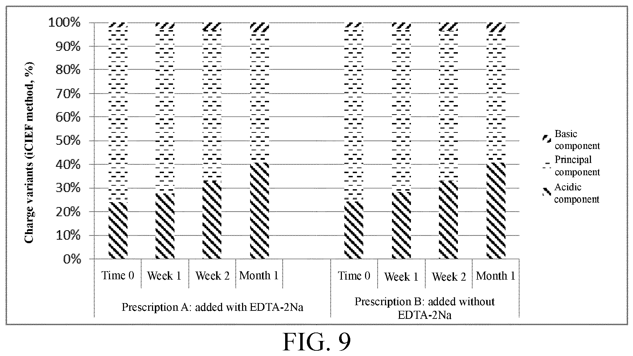

FIG. 9 is a graph showing the change in charge variants (principal component, acidic component and basic component) over time of the antibody formulations added with or without EDTA (prescriptions A and B) after storage at 40 ± 2 °C for 0 days, 1 week, 2 weeks and 1 month as determined by the iCIEF method in the antioxidant screening test described in the example.

### DETAILED DESCRIPTION

Before the present invention is described in detail, it should be understood that the present invention is not limited to the particular methodology or experimental conditions described, as such methods and conditions may vary. Further, the terms used herein are for the purpose of describing particular embodiments only and are not intended to be limiting.

### Definitions

Unless defined otherwise, all technical and scientific terms used herein have the same meaning as commonly understood by those of ordinary skill in the art. For the purposes of the present invention, the following terms are defined below.
The term "about" used in combination with a numerical value is intended to encompass the numerical values in a range from a lower limit less than the specified numerical value by 5% to an upper limit greater than the specified numerical value by 5%.
The term "and/or", when used to connect two or more options, should be understood to refer to any one of the options or any two or more of the options.
As used herein, the term "comprise" or "include" is intended to include the described elements, integers or steps, but not to exclude any other elements, integers or steps. As used herein, the term "comprise" or "include", unless indicated otherwise, also encompasses the situation where the entirety consists of the described elements, integers or steps. For example, when referring to "comprise" an antibody variable region of a particular sequence, it is also intended to encompass an antibody variable region consisting of the particular sequence.

As used herein, the term "antibody" refers to a polypeptide comprising light chain and heavy chain variable regions of immunoglobulin that specifically identify and bind to an antigen. Preferably, the antibody disclosed herein is a full-length antibody, consisting of two heavy chains and two light chains, wherein each heavy chain consists of a heavy chain variable region (abbreviated herein as VH) and a heavy chain constant region, and each light chain consists of a light chain variable region (abbreviated herein as VL) and a light chain constant region. In some embodiments, the antibody disclosed herein can also refer to an antigen binding fragment of an antibody.

The term "antibody formulation" refers to a formulation in a form that allows the biological activity of an antibody as an active ingredient to be exerted effectively and does not contain other components having unacceptable toxicity to a subject to which the formulation is to be administered. Such antibody formulations are generally sterile. Generally, the antibody formulation comprises a pharmaceutically acceptable excipient. A "pharmaceutically acceptable" excipient is an agent that can be reasonably administered to a mammal subject so that an effective dose of the active ingredient used in the formulation can be delivered to the subject. The concentration of the excipient is adapted to the mode of administration and may, for example, be acceptable for injection.
The term "anti-CD47 antibody formulation", herein also referred to as the "antibody formulation disclosed herein", means a formulation comprising an anti-CD47 antibody as an active ingredient and a pharmaceutically acceptable excipient. After the combination, the anti-CD47 antibody as an active ingredient is suitable for therapeutic or prophylactic administration to a human or non-human animal. The antibody formulation disclosed herein can be prepared, for example, as an aqueous liquid formulation, e.g., in a ready-to-use pre-filled syringe, or as a lyophilized formulation to be reconstituted (i.e., redissolved) by dissolution and/or suspension in a physiologically acceptable solution immediately prior to use. In some embodiments, the anti-CD47 antibody formulation is in the form of a liquid formulation.

A "stable" antibody formulation is the antibody in the formulation that retains an acceptable degree of physical and/or chemical stability after storage under specific conditions. Although the antibody in the antibody formulation may not maintain 100% of its chemical structure after storage for a specific period of time, the antibody formulation is considered "stable" when the antibody typically maintains about 90%, about 95%, about 96%, about 97%, about 98%, or about 99% of its structure or function after storage for a specific period of time. In some specific embodiments, the antibody aggregation or degradation or chemical modification is barely detected in the anti-CD47 antibody formulation disclosed herein during manufacture, formulation, transportation and long-term storage, resulting in little or even no loss of biological activity of the anti-CD47 antibody and exhibiting high stability. In some embodiments, the anti-CD47 antibody formulation disclosed herein substantially retains its physical and chemical stability after storage. Preferably, the liquid formulation disclosed herein can remain stable at room temperature or at 40 °C for at least 1 month and/or at 2-8 °C for at least 24 months.
A variety of analytical techniques are known in the art for determining the stability of proteins, see, e.g., Peptide and Protein Drug Delivery, 247-301, Vincent Lee Ed., Marcel Dekker, Inc., New York, N.Y., Pubs (1991) and Jones, A. Adv. Drug Delivery Rev. 10: 29-90 (1993). Stability can be determined at a selected temperature and for a selected storage time. For example, the storage time can be selected based on the expected shelf life of the formulation. Alternatively, an accelerated stability test can be adopted. In some embodiments, the stability test is performed by conducting various stress tests on the antibody formulation. These tests can represent extreme conditions that a formulated antibody formulation may encounter during manufacture, storage or transportation, and can also represent conditions that may accelerate the instability of the antibody in the antibody formulation during non-manufacture, storage or transportation. For example, the formulated anti-CD47 antibody formulation can be filled into a 5 mL glass vial for shaking stress to test the shaking/shear stability of the antibody; alternatively, the formulated anti-CD47 antibody formulation can be filled into a glass vial to test the stability of the antibody under high temperature stress.

If the formulation does not exhibit aggregation, precipitation, turbidity and/or denaturation, or exhibits very little aggregation, precipitation, turbidity, and/or denaturation after storage for a period of time, the antibody can be considered to "maintain its physical stability" in the formulation. Safety issues arise due to the aggregation of antibodies in the formulation, which can potentially lead to an increased immune response in a patient. Accordingly, there is a need to minimize or prevent the aggregation of antibodies in the formulation. Light scattering methods can be used to determine visible aggregates in the formulation. SEC can be used to determine soluble aggregates in the formulation. In addition, the stability of the formulation can be indicated by visually inspecting the appearance, color and/or clarity of the formulation, or by detecting the turbidity of the formulation by the OD_{350 nm} method, or by determining the purity of the formulation by the non-reduced CE-SDS method. In one embodiment, the stability of the formulation is measured by determining the percentage of antibody monomers in the formulation after storage at a particular temperature for a particular period of time, wherein the higher the percentage of antibody monomers in the formulation, the higher the stability of the formulation.
An "acceptable degree" of physical stability can represent at least about 92% of anti-CD47 antibody monomers detected in the formulation after storage at a specific temperature for a specific period of time. In some embodiments, an acceptable degree of physical stability represents at least about 92%, 93%, 94%, 95%, 96%, 97%, 98%, 99% of anti-CD47 antibody monomers after storage at a specific temperature for at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or longer. When assessing the physical stability, the specific temperature at which the pharmaceutical formulation is stored can be any temperature from about -80 °C to about 45 °C, e.g., at about -80 °C, about -30 °C, about -20 °C, about 0 °C, about 4-8 °C, about 5 °C, about 25 °C, about 35 °C, about 37 °C, about 40 °C, about 42 °C, or about 45 °C. For example, if at least about 92%, 93%, 94%, 95%, 96%, 97%, 98%, 99% of anti-CD47 antibody monomers are detected after storage at about 40 ± 2 °C for 1 month, the pharmaceutical formulation is considered stable. If at least about 92%, 93%, 94%, 95%, 96%, 97%, 98%, 99% of anti-CD47 antibody monomers are detected after storage at about 25 °C for 2 months, the pharmaceutical formulation is considered stable. If at least about 92%, 93%, 94%, 95%, 96%, 97%, 98%, 99% of anti-CD47 antibody monomers are detected after storage at about 5 °C for 9 months, the pharmaceutical formulation is considered stable.

If the antibody in the formulation does not exhibit significant chemical changes after storage for a period of time, the antibody can be considered to "maintain its chemical stability" in the formulation. Most of the chemical instability results form the formation of covalently modified forms of the antibody (e.g., charge variants of the antibody). Basic variants can be formed, for example, by aspartic acid isomerization, and N- and C-terminal modifications; acidic variants can be produced by deamidation, sialylation and saccharification. Chemical stability can be assessed by detecting and/or quantifying chemically altered forms of the antibody. For example, charge variants of the antibody in the formulation can be detected by cation exchange chromatography (CEX) or imaging capillary isoelectric focusing (icIEF). In one embodiment, the stability of the formulation is measured by determining the percentage change in charge variants of the antibody in the formulation after storage at a specific temperature for a specific period of time, wherein the smaller the change, the higher the stability of the formulation.
An "acceptable degree" of chemical stability can represent the percentage change in charge variants (e.g., principal component, acidic component or basic component) in the formulation of no more than 30%, e.g., 20%, after storage at a specific temperature for a specific period of time. In some embodiments, an acceptable degree of chemical stability can represent the percentage change in charge variant (acidic component) of no more than about 25%, 20%, 15%, 10%, 5%, 4%, 3%, 2% or 1% after storage at a specific temperature for at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or longer. When chemical stability is assessed, the temperature at which the pharmaceutical formulation is stored can be any temperature from about -80 °C to about 45 °C, e.g., at about -80 °C, about -30 °C, about -20 °C, about 0 °C, about 4-8 °C, about 5 °C, about 25 °C, or about 45 °C. For example, the pharmaceutical formulation can be considered stable if the percentage change in charge variant (acidic component) after storage at 5 °C for 24 months is less than about 25%, 24%, 23%, 22%, 21%, 20%, 19%, 18%, 17%, 16%, 15%, 14%, 13%, 12%, 10%, 9%, 8%, 7%, 6%, 5%, 4%, 3%, 2%, 1%, 0.5% or 0.1%. The pharmaceutical formulation can also be considered stable if the percentage change in charge variant (acidic component) after storage at 25 °C for 2 months is less than about 20%, 19%, 18%, 17%, 16%, 15%, 14%, 13%, 12%, 10%, 9%, 8%, 7%, 6%, 5%, 4%, 3%, 2%, 1%, 0.5% or 0.1%. The pharmaceutical formulation can also be considered stable if the percentage change in charge variant (acidic component) after storage at 40 °C for 1 month is less than about 25%, 24%, 23%, 22%, 21%, 20%, 19%, 18%, 17%, 16%, 15%, 14%, 13%, 12%, 10%, 9%, 8%, 7%, 6%, 5%, 4%, 3%, 2%, 1%, 0.5% or 0.1%.

The term "lyophilized formulation" refers to a composition obtained or obtainable by a freeze-drying process of a liquid formulation. Preferably, it is a solid composition having a water content of less than 5%, preferably less than 3%.
The term "reconstituted formulation" refers to a liquid formulation obtained by dissolving and/or suspending a solid formulation (e.g., a lyophilized formulation) in a physiologically acceptable solution.
As used herein, the term "room temperature" refers to a temperature from 15 °C to 30 °C, preferably from 20 °C to 27 °C, more preferably 25 °C.
"Stress conditions" refer to environments that are chemically and/or physically unfavorable to antibody proteins and may result in unacceptable destabilization of the antibody proteins. "High temperature stress" means that the antibody formulation is stored at room temperature or even higher temperature (e.g., 40 ± 2 °C) for a period of time. The stability of the antibody formulation can be tested by the high-temperature stress accelerated test.
As used herein, the term "parenteral administration" means modes of administration other than enteral and topical administration, typically by injection or infusion, including but not limited to, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. In some embodiments, the stable anti-CD47 antibody formulation disclosed herein is administered parenterally to a subject. In one embodiment, the anti-CD47 antibody formulation is administered by subcutaneous, intradermal, intramuscular, or intravenous injection to a subject.

### I. Antibody Formulation

The present invention provides a stable liquid antibody formulation comprising (i) a recombinant fully human anti-CD47 monoclonal antibody, (ii) a buffer, (iii) a stabilizer, and (iv) a surfactant, wherein the antibody formulation is at about pH 5.0-6.0. In a preferred embodiment, the liquid antibody formulation disclosed herein is in the form of an injection.

### (i) Anti-CD47 antibody

An "anti-CD47 antibody" refers to an antibody that is capable of binding to a CD47 molecule with sufficient affinity such that the antibody can be used as a therapeutic and/or prophylactic agent targeting the CD47 molecule.
The antibody disclosed herein is a recombinant fully human antibody. The terms "fully human antibody" and "human antibody" are used interchangeably herein and refer to an antibody comprising variable regions in which both framework and CDR regions are derived from human germline immunoglobulin sequences, and if the antibody comprises constant regions, the constant regions are derived from human germline immunoglobulin sequences as well. The human antibody disclosed herein can include amino acids (e.g., mutations introduced by *in vitro* random or site-directed mutagenesis or *in vivo* somatic mutation) not encoded by human germline immunoglobulin sequences. However, as used herein, the term "human antibody" is not intended to include antibodies in which the CDR sequences, derived from the germline of other mammalian species (e.g., mice), are grafted into human framework sequences. As used herein, the term "recombinant human antibody" includes all human antibodies that are prepared, expressed, produced or isolated by recombinant means. These recombinant human antibodies have variable regions in which both framework regions and CDR regions are derived from human germline immunoglobulin sequences. However, in certain embodiments, the recombinant human antibodies can be subjected to *in vitro* mutagenesis (or *in vivo* somatic mutagenesis in the case of transgenic animals using human Ig sequences), and the amino acid sequences of the VH and VL regions of the resulting recombinant antibodies, although derived from and related to human germline VH and VL sequences, do not naturally occur in a human antibody germline repertoire.
In some embodiments, the anti-CD47 antibody can specifically bind to human CD47 with a high affinity, e.g., with K_{D} of 10⁻⁷ M or less, preferably 10⁻⁹ M to 10⁻¹⁰ M as measured by biological optical interferometry, and thus mediates the efficient blocking of CD47 binding to its ligand.
In some embodiments, the anti-CD47 antibody disclosed herein comprises: a heavy chain (VH) of SEQ ID NO: 1 or a VH having at least 90% identity thereto; and a light chain variable region (VL) of SEQ ID NO 2 or a VL having at least 90% identity thereto. "Variable region" or "variable domain" is a domain in the heavy or light chain of an antibody that is involved in binding of the antibody to an antigen thereof. Generally, a heavy chain variable region (VH) and a light chain variable region (VL) can be further divided into hypervariable regions (HVRs, also known as complementarity determining regions (CDRs)) with more conservative regions (i.e., framework regions (FRs)) inserted therebetween. Each VH or VL consists of three CDRs and four FRs, arranged from amino-terminus to carboxyl-terminus in the following order: FR1, CDR1, FR2, CDR2, FR3, CDR3 and FR4.
In some embodiments, the anti-CD47 antibody disclosed herein comprises the HCDR1, HCDR2 and HCDR3 sequences of the heavy chain variable region of SEQ ID NO: 1 and LCDR1, LCDR2 and LCDR3 sequences of the light chain variable region of SEQ ID NO: 2. "Complementarity determining region", "CDR region" or "CDR" (used interchangeably herein with a "hypervariable region" (HVR)) is an amino acid region in the variable region of an antibody that is primarily responsible for binding to an epitope of an antigen. The CDRs of the heavy and light chains are generally referred to as CDR1, CDR2, and CDR3, and are numbered sequentially from the N-terminus. The CDRs located in the heavy chain variable domain of the antibody are referred to as HCDR1, HCDR2 and HCDR3, whereas the CDRs located in the light chain variable domain of the antibody are referred to as LCDR1, LCDR2 and LCDR3. Various schemes for determining the CDR sequence of a given VH or VL amino acid sequence are known in the art. For example, Kabat complementarity determining regions (CDRs) are determined based on sequence variability and are the most commonly used (Kabat et al., Sequences of Proteins of Immunological Interest, 5th Ed. Public Health Service, National Institutes of Health, Bethesda, Md. (1991)). Chothia scheme is based on the positions of structural loops (Chothia and Lesk, J. mol. biol. 196:901-917 (1987)). AbM HVRs are a compromise between Kabat HVRs and Chothia structural loops and are used by Oxford Molecular's AbM antibody modeling software. The "contact" HVRs are based on analysis of available complex crystal structures. HVRs can also be determined based on the same Kabat numbering position as the reference CDR sequences (e.g., exemplary CDRs disclosed herein).

In one embodiment, the boundaries of the CDRs of the antibody disclosed herein are determined by the Kabat scheme, e.g., as shown in Table A(1) below.

In one embodiment, the boundaries of the CDRs of the antibody disclosed herein are determined by taking Kabat, AbM, Chothia, and empirical factors into consideration. For example, as shown in Table A(2) below: HCDR1 is the sequence at positions H27-H35 in the Kabat numbering system, HCDR3 is the sequence at positions H93-H102 in the Kabat numbering system, and HCDR2 and LCDR1, LCDR2, and LCDR3 are determined by the Kabat scheme.

However, it should be noted that boundaries of the CDRs of variable regions of the same antibody based on different assignment systems may differ. That is, the CDR sequences of variable regions of the same antibody defined by different assignment systems differ. Accordingly, when it comes to defining an antibody with specific CDR sequences defined in the present invention, the scope of antibody also encompasses such antibodies whose variable region sequences comprise the specific CDR sequences, but having claimed CDR boundaries different from the specific CDR boundaries defined by the present invention due to a different protocol (e.g., different assignment system rules or their combinations) applied.

Antibodies with different specificities (i.e., different binding sites for different antigens) have different CDRs. However, although CDRs differ from antibody to antibody, only a limited number of amino acid positions within the CDRs are directly involved in antigen binding. The smallest overlapping region can be determined using at least two of the Kabat, Chothia, AbM, Contact, and North methods, thereby providing a "minimal binding unit" for antigen binding. The minimal binding unit may be a sub-portion of the CDR. As will be clear to those skilled in the art, residues in remaining portions of the CDR sequences can be determined by the structure and protein folding of the antibody. Accordingly, variants of any CDR presented herein are also considered. For example, in a variant of one CDR, the amino acid residue of the minimal binding unit may remain unchanged, while the remaining CDR residues defined by the Kabat or Chothia may be conservatively substituted.

**Table A**

| **Combinations** | **HCDR1** | **HCDR2** | **HCDR3** | **LCDR1** | **LCDR2** | **LCDR3** |
|---|---|---|---|---|---|---|
| (**1**) (**Determined according to the Kabat scheme**) | SYYWS (SEQ ID NO: 11) | YIYYSGSTNYNPSLKS (SEQ ID NO: 4) | GKTGSAA (SEQ ID NO: 12) | RASQGISRWLA (SEQ ID NO: 6) | AASSLQS (SEQ ID NO: 7) | QQTVSFPIT (SEQ ID NO: 8) |
| (**2**) (**Determined empirically**) | GSISSYYWS (SEQ ID NO: 3) | YIYYSGSTNYNPSLKS (SEQ ID NO: 4) | ARGKTGSAA (SEQ ID NO: 5) | RASQGISRWLA (SEQ ID NO: 6) | AASSLQS (SEQ ID NO: 7) | QQTVSFPIT (SEQ ID NO: 8) |

In some embodiments, the anti-CD47 antibody disclosed herein can comprise a heavy chain variable region (VH) having at least 90%, 95%, or 98% or more identity to SEQ ID NO: 1; and/or a light chain variable region (VL) having at least 90%, 95% or 98% or more identity to SEQ ID NO: 2. As used herein, the term "sequence identity" refers to the degree to which sequences are identical on a nucleotide-by-nucleotide or amino acid-by-amino acid basis in a comparison window. The "percent sequence identity" can be calculated by the following steps: comparing two optimally aligned sequences in a comparison window; determining a number of positions in which nucleic acid bases (e.g., A, T, C, G and I) or amino acid residues (e.g., Ala, Pro, Ser, Thr, Gly, Val, Leu, Ile, Phe, Tyr, Trp, Lys, Arg, His, Asp, Glu, Asn, Gln, Cys, and Met) are the same in the two sequences to give the number of matched positions; dividing the number of matched positions by the total number of positions in the comparison window (i.e., the window size); and multiplying the result by 100 to give a percent sequence identity. Optimal alignment for determining the percent sequence identity can be achieved in a variety of ways known in the art, for example, using publicly available computer software such as BLAST, BLAST-2, ALIGN, or Megalign (DNASTAR) software. Those skilled in the art can determine suitable parameters for alignment of the sequences, including any algorithms necessary to achieve optimal alignment in a full-length sequence range or target sequence region being compared.

In some embodiments, the VH sequence of the antibody disclosed herein has no more than 10, preferably no more than 5, 4 or 3 different residues as compared to SEQ ID NO: 1, preferably the different residues are conservative amino acid substitutions. In some embodiments, the VL sequence of the antibody disclosed herein has no more than 10, preferably no more than 5, 4 or 3 different residues as compared to SEQ ID NO: 2, preferably the different residues are conservative amino acid substitutions. The "conservative substitution" refers to an amino acid alteration that results in the replacement of an amino acid with a chemically similar amino acid. Conservative substitution tables providing functionally similar amino acids are well known in the art. In any one of the embodiments disclosed herein, in one preferred aspect, the conservatively substituted residue is from the conservative substitution Table X below, preferably the preferred substituted residues shown in Table X.

**Table X**

| Original residues | Exemplary substitution | Preferred conservative amino acid substitution |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Nle | Leu |
| Leu (L) | Nle; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Nle | Leu |

In some embodiments, the antibody disclosed herein is an antibody in the form of IgG4. "Antibody in the form of IgG" refers to the heavy chain constant region of the antibody belonging to the IgG form. Heavy chain constant regions of all antibodies of the same type are identical, and heavy chain constant regions of antibodies of different types are different. For example, an antibody in the form of IgG4 refers to the Ig domain of its heavy chain constant region being an Ig domain of IgG4.
In a preferred embodiment, the anti-CD47 antibody disclosed herein is the anti-CD47 monoclonal antibody ADI-26630 disclosed in Chinese Application No. CN201710759828.9 (Aug. 29, 2017), which has a heavy chain of SEQ ID NO: 9 and a light chain of SEQ ID NO: 10. In one embodiment, the anti-CD47 antibody is an IgG4 antibody produced by the recombinant expression from CHO cells and purified. Preferably, the antibody in the liquid formulation disclosed herein exhibits significant anti-tumor activity. For example, in a mouse tumor model in which human burkitt's lymphoma cells are transplanted, e.g., intraperitoneal injection of 5 mg/kg antibody once every two days for two consecutive weeks can result in a tumor growth inhibition of about 50% or more, e.g., 100%; and/or the tumor disappearance rate reaches more than 60%.

The amount of antibody or antigen binding fragment thereof in the antibody formulation disclosed herein can vary with the specific desired characteristics of the formulation, the specific environment, and the specific purpose for which the formulation is used. In some embodiments, the antibody formulation is a liquid formulation, which may contain about 1-150 mg/mL, preferably about 10-100 mg/mL, e.g., about 15 mg/mL, 20 mg/mL, 25 mg/mL, 30 mg/mL, 35 mg/mL, 40 mg/mL, 45 mg/mL, 50 mg/mL, 55 mg/mL or 60 mg/mL anti-CD47 antibody. In one embodiment, the present invention relates to a formulation having a high concentration of anti-CD47 antibody, e.g., containing 50-150 mg/mL anti-CD47 antibody. It is known in the art that such a high-concentration antibody formulation may be diluted prior to injection, for example, if a lower antibody concentration is required for a specific therapeutic or prophylactic intervention or when treating less weighted patients, including children. A suitable concentration can be 25 mg/mL or 10 mg/mL. Alternatively, the original formulation can be produced at such low concentrations.

### (ii) Buffer

Buffers are reagents that can control the pH of a solution within an acceptable range. In some embodiments, the buffer for use in the formulation disclosed herein can control the pH of the formulation disclosed herein to be about 5.0-6.0, e.g., about 5.2-5.8, preferably 5.3-5.7. In a specific embodiment, the antibody formulation disclosed herein is at about pH 5.0, pH 5.2, pH 5.4, pH 5.5, pH 5.6, pH 5.7, pH 5.8, pH 5.9 or pH 6.0, preferably at about pH 5.5. Preferably, the buffer for use in the present invention is a histidine buffer, e.g., a buffer system consisting of histidine and histidine hydrochloride.

In one embodiment, the concentration of buffer in the antibody formulation disclosed herein is about 5-100 mM, preferably about 10-50 mM, e.g., about 10-20 mM. Preferably, the buffer is about 10-20 mM histidine buffer.

### (iii) Stabilizer

Suitable stabilizers for use in the present invention can be selected from saccharides, polyols and amino acids and combinations thereof. Saccharides used as stabilizers include, but are not limited to, sucrose and trehalose. Polyols used as stabilizers include, but are not limited to, sorbitol. Amino acids used as stabilizers include, but not limited to, arginine.

In one embodiment, the liquid formulation disclosed herein comprises sucrose as a stabilizer. The amount of sucrose in the liquid formulation disclosed herein can be about 50-100 mg/mL, preferably 70-90 mg/mL, e.g., 80 mg/mL.

In one embodiment, the liquid formulation disclosed herein comprises trehalose as a stabilizer. The amount of trehalose in the liquid formulation disclosed herein can be about 50-100 mg/mL, preferably 70-90 mg/mL, e.g., 80 mg/mL.

In one embodiment, the liquid formulation disclosed herein comprises arginine as a stabilizer. The amount of arginine in the liquid formulation disclosed herein can be about 80-110 mM, particularly about 100 mM, e.g., arginine hydrochloride at about 21.1 mg/mL.

In one embodiment, the liquid formulation disclosed herein comprises sorbitol as a stabilizer. The amount of sorbitol in the liquid formulation disclosed herein can be about 10-100 mg/mL, preferably 20-70 mg/mL, e.g., 30-60 mg/mL. For example, the amount of sorbitol can be about 10 mg/mL, 15 mg/mL, 20 mg/mL, 25 mg/mL, 30 mg/mL, 35 mg/mL, 40 mg/mL, 45 mg/mL, 50 mg/mL, 55 mg/mL, 60 mg/mL, 65 mg/mL or 70 mg/mL, preferably about 40 mg/mL.

In one embodiment, the liquid formulation disclosed herein comprises a combination of sorbitol and arginine as a stabilizer. In the combination, the amount of sorbitol can be about 5-60 mg/mL, preferably 10-30 mg/mL, e.g., 10-20 mg/mL, e.g., 5 mg/mL, 10 mg/mL, 12 mg/mL, 15 mg/mL, 17 mg/mL, 20 mg/mL, 22 mg/mL or 25 mg/mL. In the combination, the amount of arginine can be about 80-110 mM, particularly about 100 mM. Preferably, the liquid formulation disclosed herein comprises about 10-20 mg/mL sorbitol and about 10-30 mg/mL arginine hydrochloride. More preferably, the liquid formulation disclosed herein comprises about 15 mg/mL sorbitol and about 21.1 mg/mL arginine hydrochloride.

### (iv) Surfactant

As used herein, the term "surfactant" refers to an organic substance with an amphiphilic structure; that is, the structure is composed of groups with opposite solubility tendencies, typically an oil-soluble hydrocarbon chain and a water-soluble ionic group.

In one embodiment, the surfactant in the liquid formulation disclosed herein is a non-ionic surfactant, e.g., alkyl poly(ethylene oxide). Specific non-ionic surfactants that can be included in the formulation disclosed herein include, for example, polysorbates such as polysorbate-20, polysorbate-80, polysorbate-60 or polysorbate-40, Plonik, and the like. In a preferred embodiment, the liquid formulation disclosed herein comprises polysorbate-20 as a surfactant.

The amount of surfactant in the antibody formulation disclosed herein can vary with the specific desired characteristics of the formulation, the specific environment, and the specific purpose for which the formulation is used. In some preferred embodiments, the formulation can comprise about 0.01-5 mg/mL, preferably about 0.1-2 mg/mL, e.g., about 0.2 mg/mL, 0.3 mg/mL, 0.4 mg/mL, 0.5 mg/mL, 0.6 mg/mL, 0.7 mg/mL, 0.8 mg/mL, 0.9 mg/mL or 1.0 mg/mL, preferably about 0.3 mg/mL polysorbate-20.

### (v) Other excipients

The antibody liquid formulation disclosed herein may or may not comprise other excipients. In one embodiment, the antibody liquid formulation disclosed herein comprises EDTA or a salt thereof. In another embodiment, the antibody liquid formulation disclosed herein does not comprise EDTA or a salt thereof. In one embodiment, the liquid antibody formulation disclosed herein added with EDTA or a salt thereof has comparable stability compared to the corresponding formulation added without EDTA or a salt thereof.

For other considerations, other excipients can also be used in the formulation disclosed herein. Such excipients include, for example, flavoring agents, antimicrobial agents, sweeteners, antistatic agents, antioxidants, gelatin, and the like. These and other known pharmaceutical excipients and/or additives suitable for use in the formulation disclosed herein are well known in the art, for example, as listed in "The Handbook of Pharmaceutical Excipients, 4th edition, edited by Rowe et al, American Pharmaceuticals Association (2003); and Remington: the Science and Practice of Pharmacy, 21st edition, edited by Gennaro, Lippincott Williams & Wilkins (2005)".

### II. Preparation of Formulation

The present invention provides a stable formulation comprising an antibody. The antibody used in the formulation disclosed herein can be prepared using techniques known in the art for the production of antibodies. For example, the antibody can be recombinantly prepared. In a preferred embodiment, the antibody disclosed herein is recombinantly prepared in CHO cells.

The use of antibodies as active ingredients in drugs is now widespread. Techniques for purifying therapeutic antibodies to pharmaceutical grade are well known in the art. For example, Tugcu et al (Maximizing productivity of chromatography steps for purification of monoclonal antibodies, Biotechnology and Bioengineering 99 (2008) 599-613) describes a monoclonal antibody three-column purification method in which ion exchange chromatography (anionic IEX and/or cationic CEX chromatography) is used after a protein A capture step. Kelley et al (Weak partitioning chromatography for anion exchange purification of monoclonal antibodies, Biotechnology and Bioengineering 101 (2008) 553-566) describes a two-column purification method in which a weak partitioning anion exchange resin is used after protein A affinity chromatography.

Generally, monoclonal antibodies recombinantly produced can be purified using conventional purification methods to provide a drug with sufficient reproducibility and moderate purity for the formulation of antibody formulations. For example, after the antibody is secreted from the recombinant expression cells into the culture medium, the supernatant from the expression system can be concentrated using a commercially available protein concentration filter, e.g., Amicon ultrafiltration device. Then the antibody can be purified by methods such as chromatography, dialysis, and affinity purification. Protein A is suitable as an affinity ligand for the purification of IgG1, IgG2 and IgG4 antibodies. Other antibody purification methods, such as ion exchange chromatography, can also be used. After the antibody with sufficient purity is obtained, a formulation comprising the antibody can be prepared according to methods known in the art.

For example, the preparation can be performed by the following steps: (1) centrifuging and clarifying the fermentation broth after the fermentation to remove impurities such as cells to obtain a supernatant, (2) capturing the antibody using affinity chromatography (for example, a protein A column with specific affinity for IgG1, IgG2 and IgG4 antibodies), (3) inactivating the virus; (4) purifying (CEX cation exchange chromatography can be adopted generally) to remove impurities in the protein; (5) filtering the virus (to reduce the virus titer by, e.g., more than 4 log10); (6) ultrafiltering/diafiltering (which can be used to replace the protein in formulation buffer to facilitate its stability and concentrate it to a suitable concentration for injection). See, e.g., B. Minow, P. Rogge, K. Thompson, BioProcess International, Vol. 10, No. 6, 2012, pp. 48-57.

### III. Analytical Method of Formulation

During the storage of antibody formulations, antibodies may undergo aggregation, degradation or chemically modification, resulting in antibody heterogeneity (including size heterogeneity and charge heterogeneity), aggregates and fragments, etc., which may affect the quality of the antibody formulations. Accordingly, it is necessary to monitor the stability of antibody formulations. Various methods are known in the art for testing the stability of antibody formulations. For example, the purity of the antibody formulation can be analyzed and the aggregation level of the antibody can be evaluated by methods such as non-reduced CE-SDS and SEC-HPLC; charge variants in the antibody formulation can be analyzed by capillary isoelectric focusing electrophoresis (cIEF), imaging capillary isoelectric focusing (iCIEF), ion exchange chromatography (IEX), and the like. In addition, the stability of the formulation can be determined quickly by visually inspecting the appearance of the formulation. The change in turbidity of the formulation can also be detected by the OD_{350 nm} method, which gives information about the amount of soluble and insoluble aggregates. In addition, the change in protein content in the formulation can be detected by the ultraviolet spectrophotometry method (UV method).

The non-reduced CE-SDS method is a method for determining the purity of monoclonal antibodies using a capillary as a separation channel. In CE-SDS, protein migration is driven by the surface charge caused by SDS binding, which is proportional to the molecular weight of the protein. Since all SDS-protein complexes have similar mass-to-charge ratios, electrophoretic separation based on the size or hydrodynamic radius of the molecules can be achieved in the molecular sieve gel matrix of the capillary. This method has been widely used to monitor the purity of denatured intact antibodies. Generally, in the non-reducing CE-SDS method, a test sample is mixed with an SDS sample buffer and iodoacetamide. Then the mixture can be incubated at 68-72 °C for about 10-15 min and cooled to room temperature before the supernatant is centrifuged for analysis. The protein migration is detected using an ultraviolet detector to obtain an electrophoresis spectrogram. The purity of the antibody formulation can be calculated as the percentage of the IgG main peak area to the sum of all peak areas. For further description of the CE-SDS method, see, e.g., Richard R. et al, Application of CE SDS gel in development of biopharmaceutical antibody-based products, Electrophoresis, 2008, 29, 3612-3620.

Size exclusion high performance liquid chromatography (SEC-HPLC method) is another important method for the standardization and quality control of monoclonal antibodies. The method mainly separates molecules based on the differences in their size or hydrodynamic radius. Antibodies can be separated in three main forms by the SEC-HPLC method: high molecular weight form (HMMS), major peak (mainly antibody monomer), and low molecular weight form (LMMS). The purity of antibody can be calculated as the percentage of the main peak area to the sum of all peak areas on the chromatogram. The percentage of antibody monomers in the formulation can be measured by the SEC-HPLC method, which gives information about the content of soluble aggregates and splices. For further description of the SEC-HPLC method, see, e.g., J. Pharm. Scien., 83:1645-1650, (1994); Pharm. Res., 11:485 (1994); J. Pharm. Bio. Anal., 15:1928 (1997); J. Pharm. Bio. Anal., 14:1133-1140 (1986). In addition, see also, e.g., R. Yang et al, High resolution separation of recombinant monoclonal antibodies by size exclusion ultra-high performance liquid chromatography (SE-UHPLC), Journal of Pharmaceutical and Biomedical Analysis (2015), http://dx.doi.org/10.1016/k.jpba.2015.02.032; and Alexandre Goyon et al, Protocols for the analytical characterization of thermal monoclonal antibodies, I-Non-denaturing chromatographic techniques, Journal of Chromatography, http://dx.doi.org/10.1016/j.jchromb.2017.05.010.

Imaging capillary isoelectric focusing (iCIEF) can be used to analyze the charge heterogeneity of monoclonal antibodies. This method can provide a quantitative distribution of charge variants. iCIEF separates molecules based on the difference in their charge in a pH gradient (apparent pI value). In iCIEF, the separation column is typically a short capillary (e.g., a 5cm×100µm silica capillary), the proteins are focused in the capillary column at high voltage, and the focus is monitored online in real time by a whole column imaging detection system operating at 280 nM. One advantage of this technique is that various charge variants of an antibody sample can be simultaneously recorded by the whole column detection system. Generally, in icIEF, the sample is mixed with urea and an icIEF buffer containing methylcellulose, pI molecular weight standards, and ampholytes. Then the absorbance at 280 nm is measured after the sample has been focused for a period of time on an iCIEF analyzer such as an iCE280 analyzer (Protein Simple, Santa Clara, CA.) equipped with an iCIEF column such as a ProtionSimple assembled iCIEF column to obtain a spectrum of the focused mAb charge variants. In the iCEIF spectrum, protein-related peaks eluted before the main peak (i.e., principal component) are classified as acidic components, while protein-related peaks eluted after the main peak are classified as basic components. The relative amounts of the principal component, acidic component and basic component can be expressed as a percentage to the total peak area. For further description of iCIEF, see, e.g., Salas-Solano O et al, Robustness of iCIEF methodology for the analysis of monoclonal antibodies: an interlaboratory study, J Sep Sci. 2012 Nov; 35(22):3124-9. doi: 10.1002/jssc.201200633. Epub 2012 Oct 15; and Dada OO et al, Characterization of acidic and basic variants of IgG1 therapeutic monoclonal antibodies based on non-denaturing IEF fractionation, Electrophoresis. 2015 Nov; 36(21-22):2695-2702. doi: 10.1002/elps.201500219. Epub 2015 Sep 18.

The charge variants of the antibody in the antibody formulation can also be determined by cation exchange high performance liquid chromatography (CEX-HPLC). In this method, peaks eluted from the CEX-HPLC column earlier than the retention time of the main peak are labeled as "acidic peaks", while those eluted from the CEX-HPLC column later than the retention time of the main peak are labeled as "basic peaks".

Accelerated stability studies can be used to check the stability of products, which facilitates the screening of stable pharmaceutical formulations. For example, formulation samples can be placed at an elevated temperature, e.g., about 40 ± 2 °C and 25 ± 2 °C for an accelerated stability study. The test indexes can include appearance, visible particles, protein content, turbidity, purity (SEC-HPLC method, non-reduced CE-SDS method) and charge variants (iCIEF method).

A shaking test can be performed to investigate the shaking/shearing stability of the formulation. For example, formulation samples are aliquoted into vials, stoppered and capped, and then set out for the shaking test, e.g., shaking at 650 r/min for 3-5 days, after which the formulations are checked for appearance, protein content, turbidity, and purity.

In addition, the efficacy or biological activity of the antibody can be detected. For example, the ability of the antibody in the formulation to bind to its antigen can be tested. Various methods are known to those skilled in the art for quantifying the specific binding of an antibody to an antigen, such as immunoassay assays, e.g., ELISA.

The anti-CD47 antibody formulation disclosed herein is stable. In one embodiment, the purity of the anti-CD47 antibody in the antibody formulation disclosed herein is at least 90%, 91%, 92%, 93%, 94%, 95%, 96%, 97%, 98%, or 99% or more after storage at about 25 °C, 37 °C, 40 °C or 45 °C for at least 1 month or 2 months, e.g., after storage at 40 ± 2 °C for 1 month, as determined by the size exclusion chromatography or non-reduced CS-SDS. In one embodiment, at least 50%, preferably at least 55%, of the anti-CD47 antibody in the antibody formulation disclosed herein is in the non-basic and non-acidic forms (i.e., the main peak or main charge form) after storage at about 25 °C, 37 °C, 40 °C or 45 °C for at least 1 month or 2 months, e.g., after storage at 40 ± 2 °C for 1 month, as determined by imaging capillary focusing.

### IV. Use of Formulation

The antibody formulation disclosed herein comprising the anti-CD47 antibody disclosed herein is useful for treating, ameliorating or preventing a variety of CD47-related diseases or disorders. As used herein, the "CD47-related disease or disorder" refers to a disease or disorder that can be treated (e.g., ameliorated) or prevented with the anti-CD47 antibody disclosed herein. Any disease or disorder that can benefit from the treatment with the antibody disclosed herein is suitable for use in the present invention.
CD47 is a pluripotent molecule that plays an important role in the tumor cell evasion from immune surveillance. Blocking the CD47 signaling pathway can effectively stimulate the phagocytosis of macrophages to tumor cells, which can be used in the tumor immunotherapy. Accordingly, the formulation disclosed herein comprising the anti-CD47 antibody disclosed herein is particularly useful for treating, ameliorating or preventing CD47-related cancers. The cancers include, for example, but are not limited to: various hematological tumors and solid tumors, such as leukemias, including acute myeloid leukemia, B-lymphocytic chronic leukemia and acute lymphocytic leukemia; non-Hodgkin's lymphoma; myeloma; leiomyosarcoma; astrocytoma; breast cancer; ovarian cancer; and glioblastoma. See, e.g., Journal of International Oncology, Nov. 2013, 40(11):817-819. In some embodiments, the antibody formulation disclosed herein is useful for treating, ameliorating or preventing CD47-related lymphomas, e.g., burkitt's lymphoma.
The present invention also provides use of the formulation disclosed herein in preparing a medicament for delivering the anti-CD47 antibody to a mammal, or for treating, preventing or ameliorating one or more of the diseases and disorders described above. Preferably, the mammal is a human.
The antibody formulation disclosed herein can be administered to a subject or a patient in a variety of pathways. For example, administration can be performed by infusion or by a syringe. Accordingly, in one aspect, the present invention provides a delivery device (e.g., a syringe) comprising the antibody formulation disclosed herein (e.g., a pre-filled syringe). The patient will receive an effective amount of the anti-CD47 antibody as the primary active ingredient, i.e., an amount sufficient to treat, ameliorate or prevent the disease or disorder of interest.
The therapeutic effect can include a reduction in physiological symptoms. The optimal effective amount and concentration of the antibody for any specific subject will depend on a variety of factors including the age, weight, health status and/or sex of the patient, the nature and extent of the disease, the activity of the specific antibody, its clearance by the body, as well as any possible other treatments administered in combination with the antibody formulation. For a specific case, the effective amount delivered can be determined within the judgment of a clinician. Depending on the indication to be treated, an effective dose can range from about 0.005 mg/kg body weight to about 50 mg/kg body weight, or from about 0.1 mg/kg body weight to about 20 mg/kg body weight. In this aspect, the use of known antibody-based drugs can provide some guidance. The dosage can be a single-dose regimen or a multi-dose regimen.

The following examples are described to assist in understanding the present invention. The examples are not intended to be and should not be interpreted in any way as limiting the protection scope of the present invention.

### Examples

In order to develop a formulation prescription for long-term stable storage of a recombinant fully human anti-cluster of differentiation 47 (CD47) monoclonal antibody injection, and to ensure that the quality of the product is controllable over its period of validity (at least 24 months), the present inventors design tests of two stages of pre-prescription and prescription screening. The materials and methods used for the tests are as follows:

### Materials and methods

### 1.1. Chemicals

| Name | Grade | Origin and brand | Catalog No. | Criteria |
|---|---|---|---|---|
| Histidine | Pharmaceutical grade | Germany, Merck | 1.04352.1000 | Ph Eur, JP, USP |
| Histidine hydrochloride | Pharmaceutical grade | Germany, Merck | 1.04354.0500 | Ph Eur, BP, JP |
| Arginine hydrochloride | Pharmaceutical grade | Germany, Merck | 1.01544.1000 | Ph Eur, BP, JP, USP |
| Sorbitol | Pharmaceutical grade | French, Roquette | H20110265 | Ch.P, Ph Eur, USP |
| Trehalose | Pharmaceutical grade | USA, Pfanstiehl | T-104-4 | USP/NF, EP, JP |
| Sucrose | Pharmaceutical grade | Germany, Merck | 1.00892.9050 | Ph Eur, USP |
| Polysorbate- 20 | Pharmaceutical grade | Germany, Merck | 8.17072.1000 | Ph Eur, JPE, NF |
| Hydrochloric acid | Pharmaceutical grade | Germany, Merck | 1.00314.2508 | Ph Eur, BP, JP, NF |

### 1.2. Instruments and equipment

| Name | Origin and brand | Model No. |
|---|---|---|
| Multi-channel micro spectrophotometer | USA, Thermo | Nanodrop8000 |
| Clarity detector | Tianjin, Tianda Tianfa | YB-2 |
| Ultraviolet spectrophotometer | Japanese, Shimadzu | UV-1800 |
| SEC-HPLC analyzer | USA, Waters | 2695 |
| Non-Reduced CE-SDS analyzer | USA, Caliper | Labchip GX Touch HT |
| icIEF analyzer | USA, Protein simple | Maurice |

### 1.3. Test items and methods

The antibody formulation was tested for the following items: (1) the appearance and the presence of visible particles; (2) the protein content in the formulation determined by the ultraviolet method (UV method); (3) the turbidity of the formulation determined by the OD_{350 nm} method; (4) the purity of the antibody formulation determined by size exclusion high performance liquid chromatography (SEC-HPLC) and expressed as the percentage of the main peak area to the sum of all peak areas; (5) the purity of the antibody formulation determined by non-reduced capillary electrophoresis-sodium dodecyl sulfate (non-reduced CE-SDS) and expressed as the percentage of the main peak area to the sum of all peak areas; and (6) charge variants in the antibody formulation determined by imaging capillary isoelectric focusing (icIEF) and expressed as the percentage of the principal component, acidic component and basic component.

### Determination of protein content

The protein content in the sample was determined using an ultraviolet spectrophotometer (model No. UV-1800, Shimadzu, Japan).

### Determination of turbidity

The absorbance of the sample at 350 nm was determined using a multi-channel microspectrophotometer (model No. Nanodrop8000, Thermo, USA) to determine the turbidity of the sample.

### Detection of visible particles

The visible particles in the sample were detected using a clarity detector (model No. YB-2, Tianda Tianfa, Tianjin) according to the method described in the National Pharmacopoeia Committee, the Pharmacopoeia of the People's Republic of China (2015 edition, volume IV General Rule 0904 "Test for Visible Particles"), Beijing, China Medical Science Press, 2015.

### Size exclusion high performance liquid chromatography (SEC-HPLC)

In the SEC-HPLC method for analyzing the antibody formulation, the following parameters can be used:
Chromatographic column: TSK-gel SuperSW mAb HR (7.8×300 mm, 4 µm) analytical column, TSK-gel SuperSW (6.0×40 mm, 4 µm) guard column
Mobile phase: 20 mmol/L phosphate buffer + 200 mmol/L NaCl, pH 6.8
Flow rate: 0.5 mL/min
Column temperature: 25 °C
Detection wavelength: 280 nm
Injection volume: 50 µL
Sample tray temperature: about 10 °C
Run time: 30 min
Loading concentration: 2.0 mg/mL

### Non-Reduced capillary electrophoresis-sodium dodecyl sulfate (CE-SDS)

The non-reduced CE-SDS assay can be performed as follows: about 2 µL of the sample (protein content: 1 mg/mL) was added to 14 µL of non-reduced sample buffer (including 700 µL of sample buffer at pH 6.5 added with 31.3 µL of 250 mM NEM (N-ethylmaleimide)), and 28 µL of ultrapure water was added, and then the mixture was mixed well, followed by incubation at 70 °C for 10 min. After the incubation, the sample was cooled to room temperature and then transferred to a 96-well plate. CE-SDS separation was performed on LabChip GXIITouch (Perkinelmer) using Protein chips. HT Antibody Analysis 200 was selected for the analytical method, and BioRad 96 HSP-96xx (Sip 4 mm) was selected for the sample tray. Protein migration was monitored by fluorescence.

### Imaging capillary isoelectric focusing (icIEF)

The icIEF detection can be performed as follows: the antibody sample was diluted (or desalted) to about 1 mg/mL. 20 µL of the sample was added to 78 µL of icIEF buffer containing urea, arginine (Protein Simple), pI marker standards (Protein Simple, Santa Clara, CA) and Pharmalytes (GE Healthcare Bio-Science, Pittsburgh, PA). An imaging capillary isoelectric focusing spectrum was generated on a Maurice C. analyzer (Protein Simple, Santa Clara, CA) using an FC-coated iCIEF column. The sample was focused for a total of 8 min and the absorbance of focused protein at 280 nm was integrated using Empower 3 software (Waters, Milford, MA).

### Example 1. Preparation and purification of anti-CD47 antibody

The anti-CD47 antibody ADI-26630 was prepared and purified according to the method described in CN201710759828.9. The antibody consists of a heavy chain sequence of SEQ ID NO: 9 and a light chain sequence of SEQ ID NO: 10, and is an IgG4 antibody. Briefly, the antibody was recombinantly expressed in CHO cells and purified. The sample used in the pre-prescription test was purified by CEX (cation exchange chromatography) to give a protein content of 12.2 mg/mL. The sample used in the prescription screening test was purified by CEX (cation exchange chromatography) to give a protein content of 15.7 mg/mL, and then concentrated by diafiltration to 100 mg/mL.

### Example 2. Pre-prescription test

### 2.1. pH effect test

### 2.1.1 Procedures

Buffers (10 mM histidine and 5% sorbitol) were prepared with the pH adjusted to 5.0, 5.5, 6.0 and 6.5, respectively. The lab-scale samples were replaced by ultrafiltration with the prepared pH buffers, respectively. After the replacement, each sample was diluted to 20 mg/mL. The diluted samples were filtered and aliquoted into vials, stoppered and capped, and then set out for the accelerated stability test at 40 °C. The specific scheme is shown in Table 1.

**Table 1. Specific scheme for pH effect test**

| Sample name | Batch No. | Test conditions | Sampling time points | Test items |
|---|---|---|---|---|
| pH5.0 | 20170527-01 | 40 ± 2 °C | Sampling at day 0, week 1, week 2 and month 1 | Appearance, protein content, turbidity and purity (SEC-HPLC |
| pH5.5 | 20170527-02 | | | |
| pH6.0 | 20170527-05 | | | |
| pH6.5 | 20170527-06 | | | method and non-reduced CE-SDS method) |

| | | | | |
|---|---|---|---|---|
| Note: after sampling at the above time points, the samples were first put into an ultra-low temperature refrigerator and frozen for later detection, and then thawed for detection as required. | | | | |

### 2.1.2 Criteria

According to the knowledge of the product and the precision of the instrument and the method, criteria for determining that the sample test indexes have not changed compared to the initial value were set to determine whether the sample has changed, as detailed in Table 2.

**Table 2. Criteria for determining that the quality of the product has not changed**

| Test items | Criteria |
|---|---|
| Appearance | Clear, colorless or yellowish liquid, free of particles, floccules and precipitates |
| Protein content (UV method) | Change rate ≤ 10% |
| Turbidity (OD_{350 nm} method) | Change rate ≤ 10% |
| Purity (SEC-HPLC method) | Change in main peak purity ≤ 1% |
| Purity (non-reduced CE-SDS method) | Change in main peak purity ≤ 2% |

### 2.1.3 Test results

### (1) Appearance

After being place at 40 ± 2°C for 1 month, the appearance of each set of samples was qualified.

### (2) Protein content

The protein content of each set of samples was unchanged at 40 ± 2°C (see Table 3).

**Table 3. Protein content results of pH effect test (UV method, mg/mL)**

| Sample name | Day 0 | 40 ± 2 °C | | |
|---|---|---|---|---|
| | | Week 1 | Week 2 | Month 1 |
| pH5.0 | 18.8 | 18.5 | 18.8 | 18.7 |
| pH5.5 | 20.4 | 20.6 | 21.0 | 20.9 |
| pH6.0 | 21.4 | 21.3 | 21.3 | 21.3 |
| pH6.5 | 20.9 | 20.7 | 21.1 | 21.1 |

### (3) Turbidity

The turbidity of each set of samples at 40 ± 2 °C was increased and the higher pH, the faster the change rate (see Table 4 and FIG. 1).

**Table 4. Turbidity results of pH effect test (OD_{350 nm} method)**

| Sample name | Day 0 | 40 ± 2 °C | | |
|---|---|---|---|---|
| | | Week 1 | Week 2 | Month 1 |
| pH5.0 | 0.043 | 0.043 | 0.047 | 0.052 |
| pH5.5 | 0.056 | 0.061 | 0.067 | 0.077 |
| pH6.0 | 0.065 | 0.073 | 0.078 | 0.101 |
| pH6.5 | 0.063 | 0.075 | 0.088 | 0.102 |

### (4) Purity

Purity (SEC-HPLC method): After being accelerated at 40 ± 2 °C for 1 month, the purities of the samples at pH 5.0, pH 5.5 and pH 6.0 were not obviously changed, and the purity of the sample at pH 6.5 was reduced, which was mainly caused by the aggregation after acceleration. With the increase of pH, the initial purity of the sample tended to decrease. (See Table 5 and FIGs. 2-3).

**Table 5. Purity results of pH effect test (SEC-HPLC method)**

| Sample name | Day 0 | 40°C±2°C | | |
|---|---|---|---|---|
| | | Week 1 | Week 2 | Month 1 |
| pH5.0 | 99.0 | 99.1 | 99.0 | 98.6 |
| pH5.5 | 98.7 | 98.9 | 98.6 | 98.3 |
| pH6.0 | 98.5 | 98.4 | 98.0 | 97.9 |
| pH6.5 | 98.3 | 97.8 | 97.4 | 96.9 |

Purity (non-reduced CE-SDS method): After being accelerated at 40 ± 2 °C for 1 month, the purity of the sample at each pH was not obviously changed. (See Table 6).

**Table 6. Purity results of pH effect test ((non-reduced CE-SDS method))**

| Sample name | Day 0 | 40°C±2°C | | |
|---|---|---|---|---|
| | | Week 1 | Week 2 | Month 1 |
| pH5.0 | 98.8 | N/A | N/A | 98.6 |
| pH5.5 | 99.0 | N/A | N/A | 98.1 |
| pH6.0 | 99.0 | N/A | N/A | 98.5 |
| pH6.5 | 98.9 | N/A | N/A | 97.7 |

| | | | | |
|---|---|---|---|---|
| Note: N/A indicates not detected or not applicable, the same below. | | | | |

The pH effect test results show that the turbidity of the sample changes faster with the increase of the pH; at pH 6.5, the purity of the sample is decreased, which results in aggregation. Based on the above results, the product has good stability at pH 5.0-6.0.

### 2.2. Surfactant effect test

### 2.2.1 Procedures

Buffers (10 mM histidine and 5% sorbitol, pH 6.0) were prepared. The lab-scale samples were replaced by ultrafiltration, and diluted to 20 mg/mL, and the diluted samples were added without polysorbate-20 and added with polysorbate-20 at a final concentration of 0.3 mg/mL, respectively. The resulting samples were filtered and aliquoted into vials, stoppered and capped, and then set out for the shaking test. The test items included appearance, protein content, turbidity and purity (SEC-HPLC method and non-reduced CE-SDS method). The specific scheme is shown in Table 7.

**Table 7. Surfactant screening test scheme**

| Sample name | Batch No. | Test conditions and sampling time points |
|---|---|---|
| Added without polysorbate | 20170527-03 | Shaking test: 25 °C, 650 r/min, away from light, sampling at day 0, day 3, and day 5 |
| Added with polysorbate-20 | 20170619 | |

### 2.2.2 Criteria

See section 2.1.2.

### 2.2.3 Test results

After being shaken at 650 r/min for 3 days, the sample added without polysorbate-20 was turbid, and was no longer tested for other items; after being shaken for 5 days, the test results of the sample added with polysorbate-20 at 0.3 mg/mL were unchanged, which indicates that polysorbate-20 can ensure the good shaking stability of the formulation. The specific results are shown in Table 8.

**Table 8. Surfactant effect test results**

| Test items | Added without polysorbate-20 | | | Added with polysorbate-20 | | |
|---|---|---|---|---|---|---|
| | Day 0 | Day 3 | Day 5 | Day 0 | Day 3 | Day 5 |
| Appearance | Qualified | Turbid | Turbid | Qualified | Qualified | Qualified |
| Visible particles | Qualified | N/A | N/A | Qualified | Qualified | Qualified |
| Protein content (UV method, mg/mL) | N/A | N/A | N/A | 20.8 | 21.1 | 20.8 |
| Turbidity (OD_{350 nm} method) | N/A | N/A | N/A | 0.062 | 0.066 | 0.067 |
| Purity (SEC-HPLC method, %) | N/A | N/A | N/A | 98.2 | 98.3 | 98.3 |
| Purity (non-reduced CE-SDS method, %) | N/A | N/A | N/A | 99.4 | N/A | 99.4 |

Based on the above results, the formulation prescription is determined to be a histidine-histidine hydrochloride buffer system, the pH is selected to be 5.5, and the surfactant is polysorbate-20, on the basis of which the following prescription screening test is preformed.

### Example 3. Prescription screening test

### 3.1 Stabilizer screening test

### 3.1.1 Procedures

A total of 4 prescriptions were designed, as detailed in Table 9. Buffers of each prescription were prepared according to Table 9, and antibody proteins were replaced by ultrafiltration into the respective formulation solution. After the replacement, proteins of each prescription were diluted to about 120 mg/mL and polysorbate-20 was added to give a final concentration of 0.3 mg/mL. The resulting samples were filtered and aliquoted into vials, stoppered and capped, and then subjected to the accelerated stability test at 40 ± 2°C and 25 ± 2 °C. The test indexes include appearance, visible particles, protein content, turbidity, purity (SEC-HPLC method and non-reduced CE-SDS method) and charge variants (iCIEF method).

**Table 9. Alternative prescription information for stabilizer screening test**

| No. | Prescription information |
|---|---|
| Prescription 1 | 0.4 mg/mL histidine, 1.5 mg/mL histidine hydrochloride, 40 mg/mL sorbitol, 0.3 mg/mL polysorbate-20, pH 5.5 |
| Prescription 2 | 0.4 mg/mL histidine, 1.5 mg/mL histidine hydrochloride, 80 mg/mL sucrose, 0.3 mg/mL polysorbate-20, pH 5.5 |
| Prescription 3 | 0.4 mg/mL histidine, 1.5 mg/mL histidine hydrochloride, 80 mg/mL trehalose, 0.3 mg/mL polysorbate-20, pH 5.5 |
| Prescription 4 | 0.4 mg/mL histidine, 1.5 mg/mL histidine hydrochloride, 21.1 mg/mL arginine hydrochloride, 0.3 mg/mL polysorbate-20, pH 5.5 |

The detailed test conditions and sampling schedule are shown in Table 10.

**Table 10. Test conditions and sampling schedule**

| Test name | Test scheme and sampling time points |
|---|---|
| Accelerated test at 40 °C | Placed at 40 ± 2 °C, sampling at day 0, week 1, week 2 and month 1 |
| Accelerated test at 25 °C | Placed at 25 ± 2 °C, and sampling at day 0, month 1 and month 2 |

### 3.1.2 Criteria

According to the knowledge of the product and the precision of the instrument and the method, criteria for determining that the sample test indexes have not changed compared to the initial value were set to determine whether the sample has changed, as detailed in Table 11.

**Table 11. Criteria for determining that the quality has not changed**

| Test indexes | Criteria |
|---|---|
| Appearance | Clear, colorless or yellowish liquid, free of particles, floccules and precipitates |
| Visible particles | Conforms to the General Rule 0904 of the *Pharmacopoeia of the People's Republic of China* (2015 edition, volume IV) |
| Protein content (UV method) | Change rate ≤ 10% |
| Turbidity (OD_{350 nm} method) | Change value ≤ 0.02 |
| Purity (SEC-HPLC method) | Change in main peak purity ≤ 1% |
| Purity (non-reduced CE-SDS method) | Change in main peak purity ≤ 2% |
| Charge variants (iCIEF method) | Change value of each component (principal component, acidic component and basic component) ≤ 2% |

### 3.1.3 Test results

### (1) Appearance and visible particles

The appearance and visible particles of the four prescriptions were all qualified after being observed at 40 ± 2 °C for 1 month, and at 25 ± 2 °C for 2 months.

### (2) Protein content

The protein content of the four prescriptions was unchanged at both 40 ± 2 °C and 25 ± 2 °C (see Table 12).

**Table 12. Protein content results of stabilizer screening test (UV method, mg/mL)**

| Sample name | Day 0 | 40 ± 2 °C | | | 25 ± 2 °C | |
|---|---|---|---|---|---|---|
| | | Week 1 | Week 2 | Month 1 | Month 1 | Month 2 |
| Prescription 1 | 120.0 | 120.0 | 123.9 | 123.4 | 126.0 | 117.1 |
| Prescription 2 | 120.8 | 124.4 | 119.7 | 118.1 | 119.4 | 115.8 |
| Prescription 3 | 120.5 | 125.5 | 121.3 | 126.0 | 126.0 | 118.4 |
| Prescription 4 | 120.2 | 125.0 | 125.7 | 127.8 | 125.7 | 120.8 |

### (3) Purity

Purity (SEC-HPLC method): After being stored at 40 ± 2 °C for 1 month, the purity of the prescription 1 (sorbitol) was not obviously changed, and the other prescriptions exceeded the proposed criteria, mainly showing an increase in the aggregates, but the increase was < 2%; after being stored at 25 ± 2 °C, the purity of all prescription was not obviously changed. See Table 13 and FIG. 4 for details.

**Table 13. Purity results of stabilizer screening test (SEC-HPLC method, %)**

| Sample name | Day 0 | 40 ± 2 °C | | | 25 ± 2 °C | |
|---|---|---|---|---|---|---|
| | | Week 1 | Week 2 | Month 1 | Month 1 | Month 2 |
| Prescription 1 | 98.0 | 97.8 | 97.8 | 97.9 | 97.8 | 97.7 |
| Prescription 2 | 98.2 | 98.1 | 98.0 | 97.1 | 97.7 | 98.0 |
| Prescription 3 | 98.3 | 97.7 | 97.8 | 97.2 | 97.7 | 97.9 |
| Prescription 4 | 98.4 | 98.3 | 98.4 | 96.8 | 98.2 | 98.2 |

Purity (non-reduced CE-SDS method): After being stored at 40 ± 2 °C, the purity of all prescriptions tended to decrease, and there was no difference among the prescriptions; after being stored at 25 ± 2 °C, the purity of all prescriptions was not obviously changed. See Table 14 and FIG. 5 for details.

**Table 14. Purity results of stabilizer screening test (non-reduced CE-SDS method, %)**

| Sample name | Day 0 | 40 ± 2 °C | | | 25 ± 2 °C | |
|---|---|---|---|---|---|---|
| | | Week 1 | Week 2 | Month 1 | Month 1 | Month 2 |
| Prescription 1 | 98.9 | N/A | 98.0 | 97.4 | 99.1 | 98.7 |
| Prescription 2 | 98.9 | N/A | 98.2 | 97.1 | 99.1 | 98.9 |
| Prescription 3 | 99.1 | N/A | 98.2 | 97.4 | 99.1 | 98.9 |
| Prescription 4 | 99.2 | N/A | 98.2 | 97.1 | 99.1 | 98.9 |

### (4) Charge variants

After being stored at 40 ± 2 °C and 25 ± 2 °C, the charge variants - the acidic component and the principal component of all prescriptions were obviously changed, while the basic component was slightly changed; the trends of all prescriptions were consistent without significant difference, and the change range was acceptable. (See Table 15 and FIGs. 6-7)

**Table 15. Charge variant results of stabilizer screening test (iCIEF method, %)**

| | | | | | | | |
|---|---|---|---|---|---|---|---|
| Components | Sample name | Day 0 | 40 ± 2 °C | | | 25 ± 2 °C | |
| | | | Week 1 | Week 2 | Month 1 | Month 1 | Month 2 |
| | Prescription 1 | 29.8 | N/A | 37.9 | 42.8 | 32.1 | 34.4 |
| Acidic | Prescription 2 | 30.3 | N/A | 36.9 | 46.2 | 30.3 | 33.7 |
| component | Prescription 3 | 30.0 | N/A | 36.3 | 44.8 | 31.5 | 32.9 |
| | Prescription 4 | 29.2 | N/A | 36.0 | 46.3 | 30.8 | 32.1 |
| | Prescription 1 | 68.0 | N/A | 58.3 | 52.8 | 65.0 | 62.5 |
| Principal | Prescription 2 | 67.5 | N/A | 59.3 | 49.7 | 66.7 | 63.2 |
| component | Prescription 3 | 67.7 | N/A | 60.0 | 51.3 | 65.8 | 64.2 |
| | Prescription 4 | 68.4 | N/A | 60.2 | 50.1 | 66.4 | 65.0 |
| | Prescription 1 | 2.3 | N/A | 3.8 | 4.3 | 2.9 | 3.1 |
| Basic | Prescription 2 | 2.3 | N/A | 3.7 | 4.1 | 2.9 | 3.1 |
| component | Prescription 3 | 2.4 | N/A | 3.7 | 3.9 | 2.7 | 2.9 |
| | Prescription 4 | 2.4 | N/A | 3.8 | 3.7 | 2.8 | 2.9 |

From the purity results of the test (SEC-HPLC method), it can be seen that sorbitol has an advantage of being more effective in inhibiting the production of protein aggregates at the high temperature of 40 °C, and there is no significant difference in the other test items; considering that arginine has been reported to reduce the viscosity of high-concentration protein products (Borwankar A U, Dear B J, Twu A, et al. Viscosity reduction of a concentrated monoclonal antibody with arginine·HCl and arginine·glutamate[J]. Industrial & Engineering Chemistry Research, 2016, 55(43): 11225-11234.), the prescription of a stabilizer combination of sorbitol and arginine is selected for the next antioxidant investigation.

### 3.2 Antioxidant test

### 3.2.1 Procedures

The samples were divided into two parts, one of which was added with EDTA-2Na to give a final concentration of 0.01 mg/mL; the other part was added without EDTA-2Na. The protein concentration of each prescription was about 100 mg/mL. The resulting samples were filtered and aliquoted into vials, stoppered and capped, and then subjected to the accelerated stability test at 40 ± 2°C, and sampled at week 1, week 2 and month 1 for detection. The test indexes include appearance, visible particles, protein content, turbidity, purity (SEC-HPLC method and non-reduced CE-SDS method) and charge variants (iCIEF method).

**Table 16. Prescription information for antioxidant screening test**

| No. | Prescription information |
|---|---|
| Prescription A | 0.4 mg/mL histidine, 1.5 mg/mL histidine hydrochloride, 15.0 mg/mL sorbitol, 21.1 mg/mL arginine, 0.3 mg/mL polysorbate-20, 0.01 mg/mL disodium edetate, pH 5.5 |
| Prescription B | 0.4 mg/mL histidine, 1.5 mg/mL histidine hydrochloride, 15.0 mg/mL sorbitol, 21.1 mg/mL arginine, 0.3 mg/mL polysorbate-20, pH 5.5 |

### 3.2.2 Criteria

See section 3.1.2.

### 3.2.3 Antioxidant test results

After being investigated at 40 ± 2 °C for 1 month, the appearance, visible particles, protein content and purity (SEC-HPLC method) of the samples of the two prescriptions were not changed; the turbidity of the samples of the two prescriptions was increased at the same change rate; the purity (non-reduced CE-SDS method) was reduced by 2.1% in 1 month; charge variants - principal component was decreased and acidic component was increased at the same change rate. See Table 17 and FIGs. 8 and 9 for details.

The data of each test index show that both prescriptions have good stability, and there is no difference between the prescriptions, that is, whether EDTA-2Na is added or not has little influence on the stability of the product. From the viewpoint of prescription simplification and safety, prescription B is selected as the final prescription of the antibody formulation.

**Table 17. Antioxidant screening test results (40 ± 2 °C)**

| Test items | | Prescription A (added with EDTA-2Na) | | | | Prescription B (added without EDTA-2Na) | | | |
|---|---|---|---|---|---|---|---|---|---|
| | | Day 0 | Week 1 | Week 2 | Month 1 | Day 0 | Week 1 | Week 2 | Month 1 |
| Appearance | | Qualified | Qualified | Qualified | Qualified | Qualified | Qualified | Qualified | Qualified |
| Visible particles | | Qualified | Qualified | Qualified | Qualified | Qualified | Qualified | Qualified | Qualified |
| Protein content (UV method, mg/mL) | | 99.3 | 97.4 | 99.0 | 95.6 | 102.9 | 100.6 | 101.6 | 95.4 |
| Turbidity (OD_{350 nm} method) | | 0.212 | 0.224 | 0.227 | 0.246 | 0.222 | 0.223 | 0.224 | 0.245 |
| Charge variants | Acidic component | 24.2 | 27.6 | 32.6 | 40.8 | 24.3 | 28.3 | 32.9 | 40.7 |
| (iCIEF method, %) | Principal | 73.7 | 69.7 | 64.0 | 55.0 | 73.7 | 69.0 | 63.6 | 55.1 |
| | Basic component | 2.0 | 2.7 | 3.4 | 4.2 | 1.9 | 2.7 | 3.4 | 4.2 |
| Purity (SEC-HPLC method, %) | | 99.4 | 99.1 | 99.1 | 98.8 | 99.3 | 99.1 | 99.1 | 98.8 |
| Purity (non-reduced CE-SDS method, %) | | 99.2 | 98.9 | 98.4 | 97.1 | 99.1 | 98.5 | 98.2 | 97.0 |

### Conclusion

Based on the above test results, the antibody protein is stable at pH 5.0-6.0; the polysorbate-20 surfactant can ensure the good shaking stability of the product; the sorbitol in the stabilizer has a good effect of being more effective in inhibiting the production of protein aggregates at a high temperature of 40 °C; the high-temperature antioxidant test shows that there is no need to added EDTA-2Na in the prescription; in addition, the addition of a proper amount of arginine can reduce the viscosity of high-concentration protein products. Thus, a preferred formulation scheme is determined to be: 100 mg/mL recombinant fully human anti-cluster of differentiation 47 (CD47) monoclonal antibody, 0.4 mg/mL histidine, 1.5 mg/mL histidine hydrochloride, 15.0 mg/mL sorbitol, 21.1 mg/mL arginine hydrochloride, 0.3 mg/mL polysorbate-20, pH 5.5.

The exemplary embodiments of the present invention have been described above. It should be understood by those skilled in the art that these contents are merely exemplary, and various other replacements, adaptations and modifications can be made within the scope of the present invention. Accordingly, the present invention is not limited to the specific embodiments listed herein.

## Claims

1. A liquid antibody formulation at about pH 5.0-6.0, e.g., at about pH 5.5, comprising
(i) an anti-CD47 antibody,
(ii) a buffer,
(iii) a stabilizer, and
(iv) a surfactant,
wherein the anti-CD47 antibody comprises:
- heavy chain VH CDR1 of GSISSYYWS (SEQ ID NO: 3) or SYYWS (SEQ ID NO: 11);
- heavy chain VH CDR2 of YIYYSGSTNYNPSLKS (SEQ ID NO: 4);
- heavy chain VH CDR3 of ARGKTGSAA (SEQ ID NO: 5) or GKTGSAA (SEQ ID NO: 12);
- light chain VL CDR1 of RASQGISRWLA (SEQ ID NO: 6);
- light chain VL CDR2 of AASSLQS (SEQ ID NO: 7); and
- light chain VL CDR3 of QQTVSFPIT (SEQ ID NO: 8).

2. The liquid antibody formulation according to claim 1, wherein the concentration of the anti-CD47 antibody in the liquid antibody formulation is about 1-150 mg/mL, preferably about 10-120 mg/mL, e.g., 20 mg/mL, more preferably about 50-120 mg/mL, e.g., about 100 mg/mL or 120 mg/mL.

3. The liquid antibody formulation according to claim 1 or 2, wherein the buffer in the liquid antibody formulation is a histidine buffer, preferably, the concentration of the buffer is about 5-100 mM, preferably about 10-50 mM, e.g., about 10 mM or 20 mM.

4. The liquid antibody formulation according to any one of claims 1 to 3, wherein the stabilizer is selected from sorbitol, sucrose, trehalose, arginine and a combination thereof.

5. The liquid antibody formulation according to any one of claims 1 to 4, comprising sorbitol as a stabilizer, preferably sorbitol in an amount of about 10-80 mg/mL, preferably about 30-50 mg/mL, e.g., 40 mg/mL.

6. The liquid antibody formulation according to any one of claims 1 to 4, comprising a stabilizer combination of sorbitol and arginine, preferably sorbitol in an amount of about 10-30 mg/mL, particularly 15 mg/mL, and arginine in an amount of 80-110 mM, particularly about 100 mM.

7. The liquid antibody formulation according to any one of claims 1 to 6, wherein the surfactant in the liquid antibody formulation is selected from polysorbate surfactants, preferably polysorbate-20.

8. The liquid antibody formulation according to any one of claims 1 to 7, wherein the concentration of the surfactant is about 0.1-1 mg/mL, preferably about 0.1-0.5 mg/mL, e.g., about 0.2 mg/mL, 0.3 mg/mL, 0.4 mg/mL or 0.5 mg/mL.

9. The liquid antibody formulation according to any one of claims 1 to 8, wherein the liquid formulation does not comprise edetic acid (EDTA) or a salt thereof.

10. The liquid antibody formulation according to claims 1 to 9, wherein the anti-CD47 antibody comprises a heavy chain variable region VH and a light chain variable region VL, wherein the heavy chain variable region comprises a sequence of SEQ ID NO: 1 or a sequence having at least 90%, 95%, 98% or 99% identity thereto, and the light chain variable region comprises a sequence of SEQ ID NO: 2 or a sequence having at least 90%, 95%, 98% or 99% identity thereto.

11. The liquid antibody formulation according to claims 1 to 10, wherein the anti-CD47 antibody is an IgG4 antibody, preferably comprising a heavy chain sequence of SEQ ID NO: 9 or a sequence having at least 90%, 95%, 98% or 99% identity thereto, and a light chain sequence of SEQ ID NO: 10 or a sequence having at least 90%, 95%, 98% or 99% identity thereto.

12. The liquid antibody formulation according to claims 1 to 11, wherein the anti-CD47 antibody is recombinantly expressed in CHO cells.

13. The liquid antibody formulation according to claims 1 to 12, wherein the liquid formulation is an injection, preferably for use in subcutaneous or intravenous injection.

14. The liquid antibody formulation according to claim 1, comprising:
(i) 90-150 mg/mL, particularly 90-120 mg/mL, anti-CD47 antibody;
(ii) about 10-20 mM histidine buffer;
(iii) about 20-60 mg/mL (e.g., 40-50 mg/mL) sorbitol, or a combination of about 5-40 mg/mL (e.g., 10-20 mg/mL) sorbitol and about 80-110 mM (e.g., about 100 mM) arginine; and
(iv) about 0.1-0.4 mg/mL (e.g., about 0.3 mg/mL) polysorbate-20;
wherein the liquid formulation is at pH 5.5 ± 0.2, preferably at about pH 5.5.

15. The liquid antibody formulation according to claim 1, comprising:
(i) about 100 mg/mL anti-CD47 antibody;
(ii) about 0.4 mg/mL histidine and about 1.5 mg/mL histidine hydrochloride;
(iii) about 15.0 mg/mL sorbitol and about 21.1 mg/mL arginine hydrochloride; and
(iv) about 0.3 mg/mL polysorbate-20;
wherein the liquid formulation is at about pH 5.5 ± 0.2, preferably at about pH 5.5.

16. The liquid antibody formulation according to any one of claims 1 to 15, wherein the formulation is stable after storage, e.g., at 2-8 °C for at least 24 months, at room temperature for at least 3 months, or at 40 °C ± 2 °C for 1 month, and preferably, has one or more of the following characteristics:
(i) the percentage of antibody monomers in the formulation is greater than 90%, preferably greater than 95%, and preferably, the increase in the aggregates is no more than 2%, as measured by SEC;
(ii) the formulation has a purity of greater than 90%, preferably greater than 95%, as measured by non-reduced CE-SDS; and
(iii) at least 50%, preferably at least 55% of the anti-CD47 antibody in the formulation is in non-basic and non-acidic forms relative to the initial value on day 0 of storage, and preferably, the increase in charge variant (acidic component) of the antibody is no more than 20%, as measured by iCIEF.

17. A solid antibody formulation obtained by curing the liquid antibody formulation according to any one of claims 1 to 16, wherein the solid formulation is, e.g., in the form of a lyophilized formulation, preferably a lyophilized powder for injection.

18. A delivery device, comprising the liquid antibody formulation according to any one of claims 1 to 16 or the solid antibody formulation according to claim 17.

19. A pre-filled syringe, comprising the liquid antibody formulation according to any one of claims 1 to 16 or the solid antibody formulation according to claim 17 for use in intravenous or intramuscular injection.

20. Use of the liquid antibody formulation according to any one of claims 1 to 16 or the solid antibody formulation according to claim 17 in the preparation of a medicament for inducing an anti-tumor or anti-cancer activity in a subject, preferably, the tumor or cancer is selected from hematological tumors and solid tumors, such as acute myeloid leukemia (AML), chronic myeloid leukemia (CML), acute lymphocytic leukemia (ALL), chronic lymphocytic leukemia (CLL), non-Hodgkin's lymphoma (NHL), multiple myeloma (MM), lymphoma, breast cancer, head and neck cancer, gastric cancer, lung cancer, esophageal cancer, intestinal cancer, ovarian cancer, cervical cancer, liver cancer, kidney cancer, pancreatic cancer, bladder cancer, colorectal cancer, neuroglioma, melanoma and other solid tumors, wherein the tumor or cancer is preferably lymphoma, e.g., burkitt's lymphoma.


SEQUENCE LISITNG

<110>  Innovent Biologics (Suzhou) Co., Ltd.

<120>  FORMULATION COMPRISING ANTI-CD47 ANTIBODY, METHOD FOR PREPARING SAME
AND USE THEREOF

<130>  PF190796PCT

<160>  12

<170>  PatentIn version 3.3

<210>  1
<211>  115
<212>  PRT
<213>  Artificial sequence

<220>
<223>  Antibody sequence

<400>  1

Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Tyr
            20                  25                  30

Tyr Trp Ser Trp Ile Arg Gln Pro Pro Gly Lys Gly Leu Glu Trp Ile
        35                  40                  45

Gly Tyr Ile Tyr Tyr Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Gly Lys Thr Gly Ser Ala Ala Trp Gly Gln Gly Thr Leu Val Thr
            100                 105                 110

Val Ser Ser
        115

<210>  2
<211>  107
<212>  PRT
<213>  Artificial Sequence

<220>
<223>  Antibody sequence

<400>  2

Asp Ile Gln Met Thr Gln Ser Pro Ser Ser Val Ser Ala Ser Val Gly
1               5                   10                  15

Asp Arg Val Thr Ile Thr Cys Arg Ala Ser Gln Gly Ile Ser Arg Trp
            20                  25                  30

Leu Ala Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Thr Val Ser Phe Pro Ile
                85                  90                  95

Thr Phe Gly Gly Gly Thr Lys Val Glu Ile Lys
            100                 105

<210>  3
<211>  9
<212>  PRT
<213>  Artificial Sequence

<220>
<223>  Antibody sequence

<400>  3

Gly Ser Ile Ser Ser Tyr Tyr Trp Ser
1               5

<210>  4
<211>  16
<212>  PRT
<213>  Artificial Sequence

<220>
<223>  Antibody sequence

<400>  4

Tyr Ile Tyr Tyr Ser Gly Ser Thr Asn Tyr Asn Pro Ser Leu Lys Ser
1               5                   10                  15

<210>  5
<211>  9
<212>  PRT
<213>  Artificial Sequence

<220>
<223>  Antibody sequence

<400>  5

Ala Arg Gly Lys Thr Gly Ser Ala Ala
1               5


<210>  6
<211>  11
<212>  PRT
<213>  Artificial Sequence

<220>
<223>  Antibody sequence

<400>  6

Arg Ala Ser Gln Gly Ile Ser Arg Trp Leu Ala
1               5                   10


<210>  7
<211>  7
<212>  PRT
<213>  Artificial Sequence

<220>
<223>  Antibody sequence

<400>  7

Ala Ala Ser Ser Leu Gln Ser
1               5


<210>  8
<211>  9
<212>  PRT
<213>  Artificial Sequence

<220>
<223>  Antibody sequence

<400>  8

Gln Gln Thr Val Ser Phe Pro Ile Thr
1               5


<210>  9
<211>  441
<212>  PRT
<213>  Artificial Sequence

<220>
<223>  Antibody sequence

<400>  9

Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Tyr
            20                  25                  30

Tyr Trp Ser Trp Ile Arg Gln Pro Pro Gly Lys Gly Leu Glu Trp Ile
        35                  40                  45

Gly Tyr Ile Tyr Tyr Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Gly Lys Thr Gly Ser Ala Ala Trp Gly Gln Gly Thr Leu Val Thr
            100                 105                 110

Val Ser Ser Ala Ser Thr Lys Gly Pro Ser Val Phe Pro Leu Ala Pro
        115                 120                 125

Cys Ser Arg Ser Thr Ser Glu Ser Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Lys Thr Tyr Thr Cys Asn Val Asp His Lys Pro Ser Asn Thr Lys
        195                 200                 205

Val Asp Lys Arg Val Glu Ser Lys Tyr Gly Pro Pro Cys Pro Pro Cys
    210                 215                 220

Pro Ala Pro Glu Ala Ala Gly Gly Pro Ser Val Phe Leu Phe Pro Pro
225                 230                 235                 240

Lys Pro Lys Asp Thr Leu Met Ile Ser Arg Thr Pro Glu Val Thr Cys
                245                 250                 255

Val Val Val Asp Val Ser Gln Glu Asp Pro Glu Val Gln Phe Asn Trp

260                 265                 270

Tyr Val Asp Gly Val Glu Val His Asn Ala Lys Thr Lys Pro Arg Glu
        275                 280                 285

Glu Gln Phe Asn Ser Thr Tyr Arg Val Val Ser Val Leu Thr Val Leu
    290                 295                 300

His Gln Asp Trp Leu Asn Gly Lys Glu Tyr Lys Cys Lys Val Ser Asn
305                 310                 315                 320

Lys Gly Leu Pro Ser Ser Ile Glu Lys Thr Ile Ser Lys Ala Lys Gly
                325                 330                 335

Gln Pro Arg Glu Pro Gln Val Tyr Thr Leu Pro Pro Ser Gln Glu Glu
            340                 345                 350

Met Thr Lys Asn Gln Val Ser Leu Thr Cys Leu Val Lys Gly Phe Tyr
        355                 360                 365

Pro Ser Asp Ile Ala Val Glu Trp Glu Ser Asn Gly Gln Pro Glu Asn
    370                 375                 380

Asn Tyr Lys Thr Thr Pro Pro Val Leu Asp Ser Asp Gly Ser Phe Phe
385                 390                 395                 400

Leu Tyr Ser Arg Leu Thr Val Asp Lys Ser Arg Trp Gln Glu Gly Asn
                405                 410                 415

Val Phe Ser Cys Ser Val Met His Glu Ala Leu His Asn His Tyr Thr
            420                 425                 430

Gln Lys Ser Leu Ser Leu Ser Leu Gly
        435                 440

<210>  10
<211>  214
<212>  PRT
<213>  Artificial Sequence

<220>
<223>  Antibody sequence

<400>  10

Asp Ile Gln Met Thr Gln Ser Pro Ser Ser Val Ser Ala Ser Val Gly
1               5                   10                  15

Asp Arg Val Thr Ile Thr Cys Arg Ala Ser Gln Gly Ile Ser Arg Trp
            20                  25                  30

Leu Ala Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Thr Val Ser Phe Pro Ile
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  11
<211>  5
<212>  PRT
<213>  Artificial Sequence

<220>
<223>  Antibody sequence

<400>  11

Ser Tyr Tyr Trp Ser
1               5

<210> 12
<211> 7
<212> PRT
<213> Artificial Sequence

<220>
<223> Antibody sequence

<400> 12

Gly Lys Thr Gly Ser Ala Ala
1               5